# EUROPEAN PATENT SPECIFICATION

(11) **EP 4 196 191 B1**
(45) Date of publication and mention of the grant of the patent: **17.09.2025**
(21) Application number: 21763481.5
(22) Date of filing: 12.08.2021
(51) Int. Cl.: A61M 5/14, A61M 39/24, A61M 39/20, A61M 39/08, A61M 5/31, A61M 5/38, A61M 39/00

(54) **PRIME TUBE CONFIGURATIONS FOR SYRINGE**
PRIME-ROHR-KONFIGURATIONEN FÜR SPRITZE
CONFIGURATIONS DE TUBE AMORCE POUR SERINGUE

(30) Priority: 13.08.2020 US 202063065095 P
(43) Date of publication of application: 21.06.2023
(73) Proprietor: Bayer HealthCare LLC, Whippany, NJ 07981 (US)
(72) Inventor: COWAN, Kevin, Allison Park, PA 15101 (US); SPOHN, Michael, East Fenelton, PA 16034 (US); UBER, Arthur III, Pittsburgh, PA 15208 (US); HAURY, John, Sewickley, PA 15143 (US); KENT, Joseph, Pittsburgh, PA 15206 (US)
(74) Representative: BIP Patents
(86) International application number: PCT/US2021/045689
(87) International publication number: WO 2022/036058

(56) References cited:
- US-A- 5 318 520
- US-A1- 2010 063 445
- US-A1- 2014 374 353

## Description

### BACKGROUND OF THE DISCLOSURE

### Field of the Disclosure

The present disclosure relates generally to prime tubes for syringes and/or pumps, such as syringes used in a fluid injector system for injecting a contrast media for a contrast enhanced imaging procedure. Specifically, the present disclosure relates to prime tubes for use in priming and/or purging air from a reservoir after filling the reservoir and prior to performance of an injection protocol where the prime tubes assist in determining when the air is purged from the reservoir.

### Description of Related Art

Powered fluid injector systems are widely used in medical imaging procedures such as angiography, computed tomography (CT) and nuclear magnetic resonance (NMR)/magnetic resonance imaging (MRI). Such injector systems provide precision and accuracy of fluid delivery beyond that which is achievable with a manual syringe, and furthermore may offer many safety features to prevent harm to the patient during an injection procedure.

During some injection procedures, it is imperative that no air be injected into the patient. Air may be present in the syringes or reservoir of a fluid injector system as packaged from the manufacturer. Additionally, air may accumulate in the syringes or reservoirs during automatic or manual filling of the syringes from one or more bulk fluid sources. Any air present in the volume must be purged prior to an injection procedure to avoid causing an air embolism.

For example, after the syringe is filled with fluid, a tubing set may be connected to the discharge outlet of the syringe, and the system may be actuated to prime the system by ejecting the air through the tubing, until the syringe and tubing are filled only with air. While this technique may be effective in purging air from the tubing connected to the syringe, dispensing fluid from the end of the tubing may cause contamination or fouling of system components and may lead to spills in the injection suite that may be a safety hazard and must be cleaned. Furthermore, in some cases it might not be clear to the technologist when the syringe has been fully primed, for example, visualization of air in the syringe may be difficult in low light or at distances, potentially leading to the assumption that a syringe is primed.

Therefore, new prime tube configurations that retain the priming fluids and are readily visualized to ensure priming before an injection are needed.

US 2010/063445 A1 discloses a priming indicator for a fluid infusion system including a luer cap or other component of the infusion system having an indicator surface covered by a membrane. The membrane exhibits a first visual characteristic, such as being opaque, when dry and exhibits a second characteristic, such as becoming less opaque, when wet. Once the membrane becomes wet, indicia on the surface, which may be provided on a rod at least partially covered by the membrane, becomes visible, thereby indicating an intravenous tube to which the luer cap is secured has been primed or is nearly primed. The indicator may alternately be employed at an upstream end of an infusion set, such as at the port of a medical bag providing a supply of fluid, to indicate a low level of fluid in the medical bag.

### SUMMARY OF THE DISCLOSURE

In view of the foregoing, there exists a need for devices, systems, and methods for improved priming of syringes and fluid injector systems. Embodiments of the present disclosure are directed to a prime tube for use with a fluid injector. The prime tube includes a sidewall defining an internal chamber having an expandable volume, a connecter associated with a proximal end of the sidewall and configured to reversibly engage an outlet of a fluid reservoir containing a medical fluid, and a closure associated with a distal end of the sidewall, the closure permeable to air and substantially impermeable to the medical fluid. The expandable volume of the internal chamber is configured to increase as the medical fluid enters the internal chamber.

In some embodiments, the sidewall includes at least one bellows. Each of the at least one bellows is configured to transition from a contracted state to an expanded state in response to an increase in fluid pressure within the internal chamber. The volume defined by the internal chamber is greater with the at least one of the at least one bellows in the expanded state than in the contracted state. In some embodiments, the at least one bellows is stable in both the contracted state and the expanded state. An axial length of the sidewall may be greater with the at least one bellows in the expanded state than in the contracted state.

In some embodiments, the sidewall includes an elastomeric material to expand the expandable volume in response to increased fluid pressure within the internal chamber.

In some embodiments, the sidewall may be configured to transition from a contracted state to an expanded state in response to an increase in fluid pressure within the internal chamber. In the contracted state, a distal portion of the sidewall is rolled over a proximal portion of the sidewall. In the expanded state, the distal portion of the sidewall is at least partially unrolled from the proximal portion of the sidewall. The volume defined by the internal chamber is greater with the sidewall in the expanded state than in the contracted state. In some embodiments, in the contracted state, an inner surface of the distal portion of the sidewall faces an inner surface of the proximal portion of the sidewall. In some embodiments, at least a portion of the sidewall is configured to invert in response to an increase in fluid pressure within the internal chamber to increase the volume of the internal chamber.

In some embodiments, the sidewall may be in a flaccid or bent configuration in the absence of fluid flow through the internal chamber and in a rigid, extended configuration when fluid flows into the internal chamber.

In some embodiments, the sidewall may be in a rolled, coiled configuration in the absence of fluid flow through the internal chamber and in an unrolled, extended configuration when fluid flows into the internal chamber.

In some embodiments, the prime tube may further include a check valve associated with the proximal end of the sidewall and configured to prevent fluid flow out of the proximal end. In some embodiments, the closure may include a high crack pressure valve.

In some embodiments, the closure includes a porous material. For example, the closure may define at least one aperture having a cross-sectional area sized to allow passage of air and substantially prohibit passage of a medical fluid through the at least one aperture.

In some embodiments, the at least one aperture may be configured to emit an audible sound when air flows through the at least one aperture.

Other embodiments of the present disclosure, not according to the claimed invention, are directed to a prime tube for use with a fluid injection. The prime tube includes a sidewall defining an internal chamber, a connecter associated with a proximal end of the sidewall and configured to reversibly engage an outlet of a fluid reservoir containing a medical fluid, and a shuttle member configured to slide within the internal chamber in response to the medical fluid flowing into the internal chamber. The sidewall may be rigid so as to not deform in response to fluid pressure below a predetermined threshold. In some embodiments, at least one of the shuttle member and the sidewall defines an air passageway configured to allow air to flow distally past the shuttle member without causing the shuttle member to slide within the internal chamber. In some embodiments, the shuttle member may include a plug configured to form an interference fit with the outlet of the fluid reservoir such that the plug is dislodged from the outlet of the fluid reservoir at a predetermined fluid pressure. The plug may have an outside diameter sufficiently large to prevent a patient administration line from being attached to the outlet of the fluid reservoir while the plug is lodged in the outlet.

The prime tube may include a cap having a proximal end configured to engage the outlet of the fluid reservoir and a distal end configured to engage the shuttle member. The shuttle member may be configured to be at least partially recessed within the outlet of the fluid reservoir prior to a priming operation.

In some examples, the prime tube further includes at least one engagement feature on the prime tube configured to retain the sidewall to the outlet of the fluid reservoir. The shuttle member, in an initial position prior to a priming operation, may lock the at least one engagement feature to the outlet of the fluid reservoir to prohibit removal of the prime tube from the fluid reservoir when the shuttle member is in the initial position. Upon distal movement of the shuttle member within the internal chamber to a second primed position, the at least one engagement feature may be unlocked from the outlet of the fluid reservoir to allow removal of the prime tube. In some embodiments, the shuttle member may include a tip configured to extend distally from a distal end of the prime tube when the shuttle member is moved to the second primed position.

In some examples the shuttle member includes a porous material permeable to air and impermeable to the medical fluid. For example, the shuttle member may define at least one aperture having a cross-sectional area sized to allow passage of air and substantially prohibit passage of a medical fluid.

In some examples, the sidewall includes at least one indicator to indicate a distance traveled by the shuttle member corresponding to a fluid fill level of the internal chamber of the prime tube. In some embodiments, the sidewall is at least partially translucent or transparent such that the shuttle member is visible through the sidewall.

Other embodiments of the present disclosure are directed to a prime tube for use with a fluid injector system. The prime tube includes a sidewall defining an internal chamber having an expandable volume, and a connecter associated with a proximal end of the sidewall and configured to reversibly engage an outlet of a fluid reservoir containing a medical fluid. The sidewall is configured to transition from a contracted state to an expanded state in response to an increase in fluid pressure within the internal chamber. In the contracted state, a distal portion of the sidewall is rolled over a proximal portion of the sidewall. In the expanded state, the distal portion of the sidewall is at least partially unrolled from the proximal portion of the sidewall. The volume defined by the internal chamber is greater with the sidewall in the expanded state than in the contracted state. In the contracted state, an inner surface of the distal portion of the sidewall may face an inner surface of the proximal portion of the sidewall.

Other embodiments of the present disclosure are directed to a fluid injector system including at least one fluid reservoir configured for injecting a medical fluid and a prime tube. The prime tube includes a sidewall defining an internal chamber having an expandable volume, a connecter associated with a proximal end of the sidewall and configured to reversibly engage an outlet of the fluid reservoir. The system further includes at least one processor programmed or configured to determine a priming status of the prime tube.

In some embodiments, the at least one processor is programmed or configured to determine the priming status of the prime tube based on a measured fluid pressure in at least one of the prime tube and the fluid reservoir. For example, in some embodiments, the fluid injector system may further include an actuator for injecting the medical fluid from the at least one reservoir and the at least one processor may be programmed or configured to determine the priming status of the prime tube based on a measured current draw of the actuator. The at least one processor may be programmed or configured to determine the priming status of the prime tube based on a force reading on a motor of the fluid injector associated with delivering a fluid from the fluid reservoir.

In certain embodiments, the at least one processor may be programmed or configured to determine the priming status of the prime tube based on at least one of expansion of the prime tube and a shape change of the prime tube. In certain embodiments, the at least one processor may be programmed or configured to determine the priming status of the prime tube based on a sound emitted from the priming tube. In some embodiments, the sidewall includes an elastomeric material that expands the expandable volume in response to increased fluid pressure within the internal chamber.

In some embodiments, the sidewall may include at least one bellows. Each of the at least one bellows is configured to transition from a contracted state to an expanded state in response to an increase in fluid pressure within the internal chamber. The expandable volume defined by the internal chamber is greater with the at least one of the at least one bellows in the expanded state than in the contracted state.

In some embodiments, the sidewall is configured to transition from a contracted state to an expanded state in response to an increase in fluid pressure within the internal chamber. In the contracted state, a distal portion of the sidewall is rolled over a proximal portion of the sidewall. In the expanded state, the distal portion of the sidewall is at least partially unrolled from the proximal portion of the sidewall. The expandable volume defined by the internal chamber is greater with the sidewall in the expanded state than in the contracted state.

In some embodiments, the sidewall is in a rolled, coiled configuration in the absence of fluid flow through the internal chamber and in an unrolled, extended configuration when fluid flows through the internal chamber.

Other embodiments of the present disclosure, not according to the invention, are directed to a fluid injector system including at least one fluid reservoir configured for injecting a medical fluid, and a prime tube. The prime tube includes a sidewall defining an internal chamber, a shuttle member slidable within the internal chamber, and a connecter associated with a proximal end of the sidewall and configured to reversibly engage an outlet of the fluid reservoir. The fluid injector system further includes at least one processor programmed or configured to determine a priming status of the prime tube. The at least one processor may be programmed or configured to determine the priming status based on a position of the shuttle member within the internal chamber.

In some examples, the sidewall of the prime tube includes at least one indicator corresponding to a fluid fill level of the internal chamber and the at least one processor may be programmed or configured to determine the priming status based on a position of the shuttle member relative to the at least one indicator.

In some examples, the prime tube further includes a cap having a proximal end configured to engage the outlet of the fluid reservoir and a distal end configured to engage the shuttle member. The shuttle member may be configured to be at least partially recessed within the outlet of the fluid reservoir prior to a priming operation. In some embodiments, the prime tube further includes at least one engagement feature on the prime tube configured to retain the sidewall to the outlet of the fluid reservoir. The shuttle member, in an initial position prior to a priming operation, locks the at least one engagement feature to the outlet of the fluid reservoir to prohibit removal of the prime tube from the fluid reservoir when the shuttle member is in the initial position. Upon distal movement of the shuttle member within the internal chamber to a second primed position, the at least one engagement feature is unlocked from the outlet of the fluid reservoir to allow removal of the prime tube.

Further details and advantages of the various examples described in detail herein will become clear upon reviewing the following detailed description of the various examples in conjunction with the accompanying drawing figures.

### BRIEF DESCRIPTION OF THE DRAWINGS

**FIG. 1** is a perspective view of a fluid injector system according to an embodiment of the present disclosure;
**FIG. 2A** is a schematic view of a fluid injector system in accordance with an embodiment of the present disclosure;
**FIG. 2B** is a partial schematic view of the fluid injector system of **FIG. 2A****,** shown with a prime tube attached to each syringe (various components of **FIG. 2A** are not shown in **FIG. 2B** for clarity);
**FIG. 3** is a schematic view of a prime tube of **FIG. 2B** according to an embodiment of the present disclosure;
**FIG. 4A** is a perspective view of a prime tube according to an embodiment of the present disclosure;
**FIG. 4B** is an exploded view of the prime tube of **FIG. 4A****;**
**FIG. 4C** is a side cross-sectional view of the prime tube of **FIG. 4A** in a contracted state;
**FIG. 4D** is a side cross-sectional view of the prime tube of **FIG. 4A** in an expanded state;
**FIG. 4E** is a graph of pressure over time for the prime tube of **FIG. 4A****;**
**FIG. 5A** is a perspective view of a prime tube according to an embodiment of the present disclosure;
**FIG. 5B** is an exploded view of the prime tube of **FIG. 5A****;**
**FIG. 5C** is a side cross-sectional view of the prime tube of **FIG. 5A** in a contracted state;
**FIG. 5D** is a side cross-sectional view of the prime tube of **FIG. 5A** in an expanded state;
**FIG. 5E** is a graph of pressure over time for the prime tube of **FIG. 5A****;**
**FIG. 6A** is a perspective view of a prime tube according to an embodiment of the present disclosure;
**FIG. 6B** is an exploded view of the prime tube of **FIG. 6A****;**
**FIG. 6C** is a side cross-sectional view of the prime tube of **FIG. 6A** in a contracted state;
**FIG. 6D** is a side cross-sectional view of the prime tube of **FIG. 6A** in an expanded state;
**FIG. 6E** is a graph of pressure over time for the prime tube of **FIG. 6A****;**
**FIG. 7A** is a perspective view of a prime tube according to an embodiment of the present disclosure;
**FIG. 7B** is an exploded view of the prime tube of **FIG. 7A****;**
**FIG. 7C** is a side cross-sectional view of the prime tube of **FIG. 7A** in a contracted state;
**FIG. 7D** is a side cross-sectional view of the prime tube of **FIG. 7A** in an expanded state;
**FIG. 7E** is a graph of pressure over time for the prime tube of **FIG. 7A-7D****;**
**FIG. 8A** is a perspective view of a prime tube according to an embodiment of the present disclosure;
**FIG. 8B** is an exploded view of the prime tube of **FIG. 8A****;**
**FIG. 8C** is a side cross-sectional view of the prime tube of **FIG. 8A** in an initial state;
**FIG. 8D** is a side cross-sectional view of the prime tube of **FIG. 8A** in a primed state;
**FIG. 8E** is a graph of pressure over time for the prime tube of **FIG. 8A****;**
**FIG. 9A** is a perspective view of a prime tube according to an embodiment of the present disclosure;
**FIG. 9B** is an exploded view of the prime tube of **FIG. 9A****;**
**FIG. 9C** is a side cross-sectional view of the prime tube of **FIG. 9A** in an initial state;
**FIG. 10A** is a perspective view of a prime tube according to an embodiment of the present disclosure;
**FIG. 10B** is an exploded view of the prime tube of **FIG. 10A****;**
**FIG. 10C** is a side cross-sectional view of the prime tube of **FIG. 10A****;**
**FIG. 11A** is a perspective view of a prime tube according to an embodiment of the present disclosure;
**FIG. 11B** is an exploded view of the prime tube of **FIG. 11A****;**
**FIG. 11C** is a side cross-sectional view of the prime tube of **FIG. 11A** in an initial state;
**FIG. 11D** is a side cross-sectional view of the prime tube of **FIG. 11A** in a primed state;
**FIG. 12A** is a perspective view of a prime tube according to an embodiment of the present disclosure;
**FIG. 12B** is an exploded view of the prime tube of **FIG. 12A****;**
**FIG. 12C** is a side cross-sectional view of the prime tube of **FIG. 12A** in an initial state;
**FIG. 12D** is a side cross-sectional view of the prime tube of **FIG. 12A** in a primed state;
**FIG. 13** is a side cross-sectional view of a prime tube according to an embodiment of the present disclosure;
**FIG. 14A** is a side view of a prime tube according to an embodiment of the present disclosure;
**FIG. 14B** is a side view of the prime tube of **FIG. 14A** in a contracted state;
**FIG. 14C** is a side cross-sectional view of the prime tube of **FIG. 14A** in an expanded, primed state;
**FIG. 15A** is a side view of a prime tube in an initial state according to an embodiment of the present disclosure; and
**FIG. 15B** is a side view of the prime tube of **FIG. 15A** in a primed configuration.

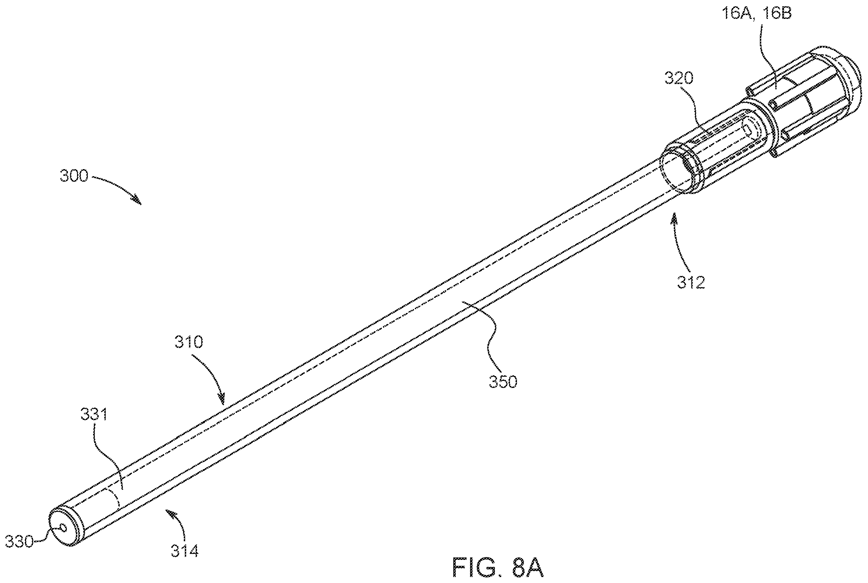

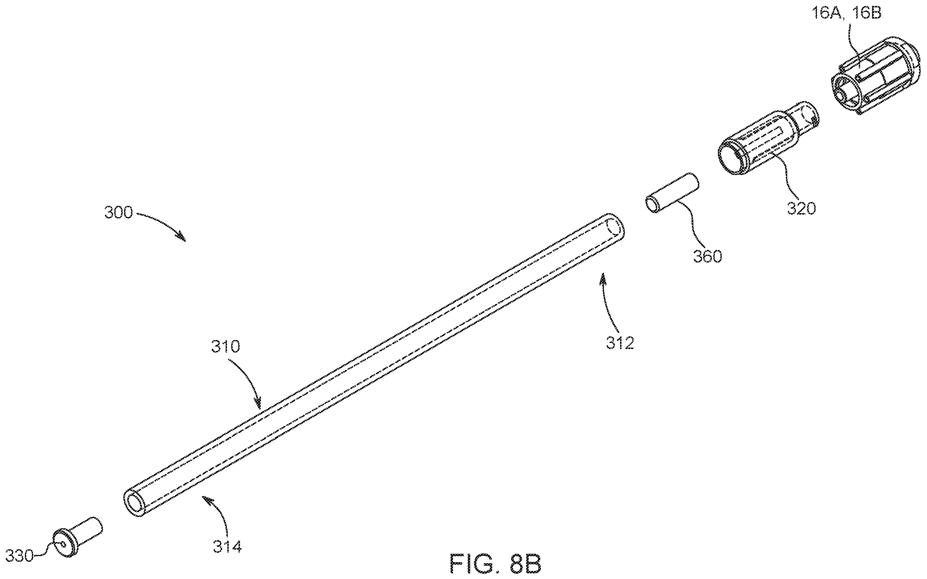

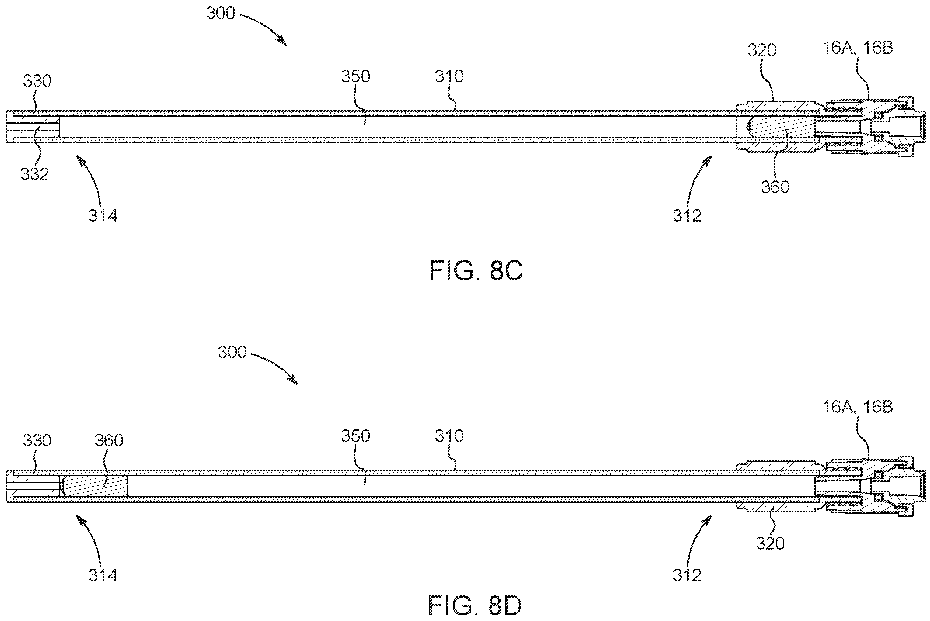

Referring to the drawings in which like reference characters refer to like parts throughout the several views thereof, the present disclosure is generally directed to a prime tube for use with a syringe of a fluid injector system. The embodiments shown in Fig. 8A-15B describe configurations not according to the claimed invention.

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures. Spatial or directional terms, such as "left", "right", "inner", "outer", "above", "below", and the like, are not to be considered as limiting as the invention can assume various alternative orientations. As used herein, the singular form of "a", "an", and "the" include plural referents unless the context clearly dictates otherwise. All numbers used in the specification and claims are to be understood as being modified in all instances by the term "about". The terms "approximately", "about", and "substantially" mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

It is also to be understood that the specific devices and processes illustrated in the attached drawings, and described in the following specification, are simply exemplary examples of the disclosure. Hence, specific dimensions and other physical characteristics related to the examples disclosed herein are not to be considered as limiting.

When used in relation to a component of a fluid delivery system such as a fluid reservoir, a syringe, an air suspension apparatus, or a fluid line, the term "distal" refers to a portion of said component nearest to a patient. When used in relation to a component of an injector system such as a fluid reservoir, a syringe, an air suspension apparatus, or a fluid line, the term "proximal" refers to a portion of said component nearest to the injector of the injector system (i.e. the portion of said component farthest from the patient). When used in relation to a component of a fluid delivery system such as a fluid reservoir, a syringe, an air suspension apparatus, or a fluid line, the term "upstream" refers to a direction away from the patient and towards the injector of the injector system. For example, if a first component is referred to as being "upstream" of a second component, the first component is located nearer to the injector than the second component is to the injector. When used in relation to a component of a fluid delivery system such as a fluid reservoir, a syringe, an air suspension apparatus, or a fluid line, the term "downstream" refers to a direction towards the patient and away from the injector of the fluid delivery system. For example, if a first component is referred to as being "downstream" of a second component, the first component is located nearer to the patient than the second component is to the patient. The terms "first", "second", and the like are not intended to refer to any particular order or chronology, but refer to different conditions, properties, or elements. The term "at least" is synonymous with "greater than or equal to". The term "not greater than" is synonymous with "less than or equal to".

It is to be understood that the disclosure may assume alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the examples disclosed herein are not to be considered as limiting.

While the devices, systems, and methods described herein are generally discussed in the context of an angiography (CV) injection system, other pressurized injection protocols, such as computed tomography (CT), ultrasound, positron emission tomography (PET), and magnetic resonance imaging (MRI) may also incorporate the various embodiments of the prime tubes described herein. Further, while many of the embodiments or prime tubes described herein are detailed in reference to a syringe or syringe based fluid injector, it should be understood that other powered injection systems, such as those including pumps such as a peristaltic pump, which require priming of a reservoir and/or a fluid tube set can be primed using various embodiments of the prime tubes herein.

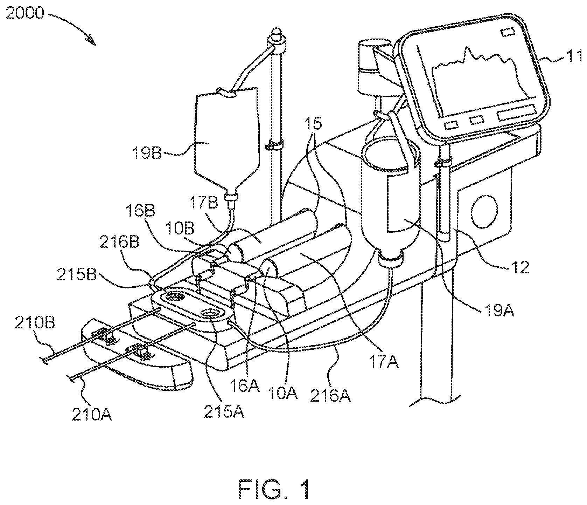

Referring to the drawings in which like reference characters refer to like parts throughout the several views thereof, the present disclosure is generally directed to prime tubes for syringes of fluid injector systems. Referring first to **FIG. 1****,** an embodiment of a dual syringe angiography injector system **2000** is illustrated. The angiography injector system **2000** is configured for injection of two medical fluids through a first fluid path **210A** for a medical fluid, such as an imaging contrast media for an angiography injection procedure, and a second fluid path **210B** for a flushing fluid, such as saline or Ringer's lactate. The fluid paths **210A, 210B** may be connected to respective outlets **16A, 16B,** e.g., nozzles, of syringes **10A, 10B.** The dual syringe angiography injector system **2000** may include an injector housing **12** having two syringe ports **15** configured to engage the syringes **10A, 10B.** In some embodiments, the syringes **10A, 10B** may be retained within corresponding pressure jackets **17A, 17B** for example to prevent pressure-induced swelling and potential bursting of the syringes **10A, 10B.**

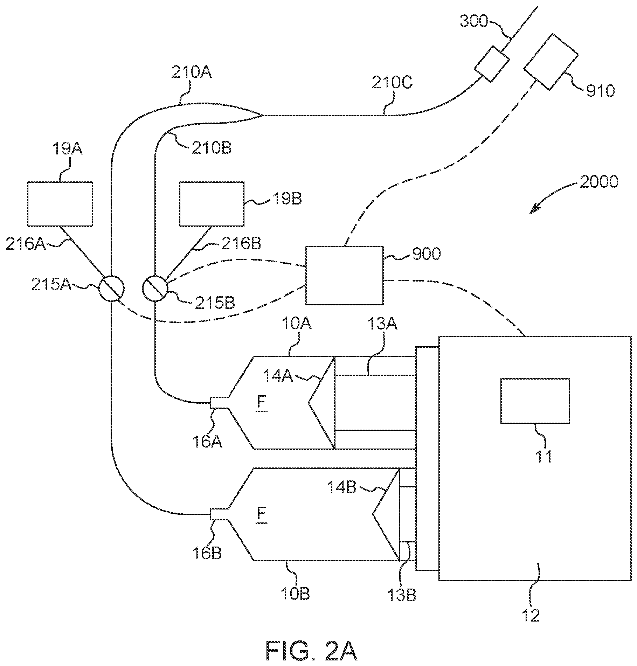

The fluid injector system **2000** may further include at least one graphical user interface (GUI) **11** through which an operator can view and control the status of an injection procedure. The **GUI 11** may be in operative communication with a controller **900** (see **FIGS. 2A-3**) which sends and receives commands between and receives input from the GUI **11** and fluid injector system **2000.**

With continued reference to **FIG. 1****,** the dual syringe angiography injector system **2000** may further include bulk fluid containers **19A** and **19B** for filling and refilling the respective syringes **10A, 10B** with imaging contrast media and flushing fluid, respectively. The bulk fluid containers **19A** and **19B** may be in selective fluid communication with the syringes **10A, 10B** via respective bulk fluid paths **216A** and **216B** and bulk fluid valves **215A** and **215B.**

Further details and examples of suitable nonlimiting powered injector systems, including syringes, pressure jackets and pressure jacket retention mechanisms, tubing, shut-off valves, controllers, and air detectors, are described in U.S. Patent Nos. 5,383,858; 7,553,294; 7,666,169; 8,945,051; 10,022,493; and 10,507,319, and International PCT Application Nos. PCT/US2013/061275; PCT/US2018/034613; PCT/US2020/049885; PCT/US2021/035273; PCT/US2021/029963; PCT/US2021/018523; PCT/US2021/037623; PCT/US2021/037574; and PCT/US2021/045298.

Referring now to **FIG. 2A****,** a schematic diagram of the fluid injector system **2000** shown in **FIG. 1** is illustrated. The injector system **2000** includes a piston **13A, 13B** respectively associated with each of the syringes **10A, 10B** and their corresponding pressure jackets **17A, 17B** (see **FIG. 1**). Each of the pistons **13A, 13B** is configured to drive a respective plunger **14A, 14B** within a barrel of the respective syringe **10A, 10B.** The controller **900** is operatively associated with the injector system **2000,** for example to activate the pistons **13A, 13B** to reciprocatively move the plungers **14A, 14B** within the syringes **10A, 10B** and thereby execute and halt an injection procedure. In a corresponding peristaltic pump system, the controller **900** would be configured to operate the rotors of the corresponding peristaltic pump. In particular, the controller **900** may include at least one processor programmed or configured to actuate the pistons **13A, 13B** and various other components of the injector system **2000,** such as one or more valves **215A, 215B,** to take in and deliver medical fluids according to a programmed protocol for an injection procedure. The controller **900** may include computer readable media, such as memory, on which one or more injection protocols may be stored for execution by the at least one processor.

The controller **900** may be programmed or configured to execute a filling operation during which the piston **13A, 13B** associated with each syringe **10A, 10B** is withdrawn toward a proximal end of the syringe **10A, 10B** to draw injection fluid **F** (e.g. imaging contrast media and flushing fluid) into the syringe **10A, 10B** from the bulk fluid containers **19A, 19B.** During such a filling operation, the controller **900** may be programmed or configured to selectively actuate the bulk fluid valves **215A** and **215B** to establish fluid communication between the respective syringes **10A, 10B** and the bulk fluid containers **19A, 19B** via the bulk fluid paths **216A** and **216B** to control filling of syringes **10A, 10B** with an appropriate injection fluid **F.**

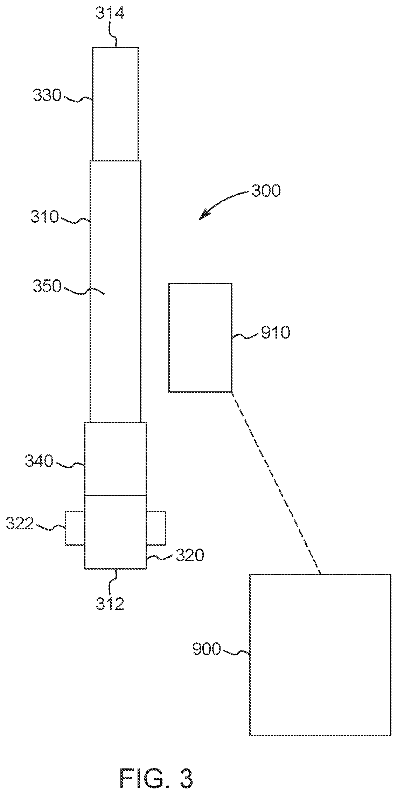

The controller **900** may be programmed or configured to execute a priming/purging operation to remove any air from the syringes **10A, 10B** upon completion of the filling operation. Specific details of the priming/purging operation will be described herein in connection with the various embodiments of the prime tubes **300** shown in **FIGS. 3-15B****.**

After the filling operation and priming operation, the controller **900** may be programmed or configured to execute a delivery operation during which the piston **13A, 13B** associated with one or both of the syringes **10A, 10B** is moved toward a distal end of the syringe to inject injection fluid **F** into the first fluid path **210A** and the second fluid path **210B,** respectively. The controller **900** may be programmed or configured to selectively actuate the bulk fluid valves **215A** and **215B** to establish fluid communication between the syringes **10A, 10B** and the patient, via the fluid paths **210A, 210B.** The first fluid path **210A** and the second fluid path **210B** ultimately merge into a patient fluid line **210C** in fluid communication with the vasculature of the patient.

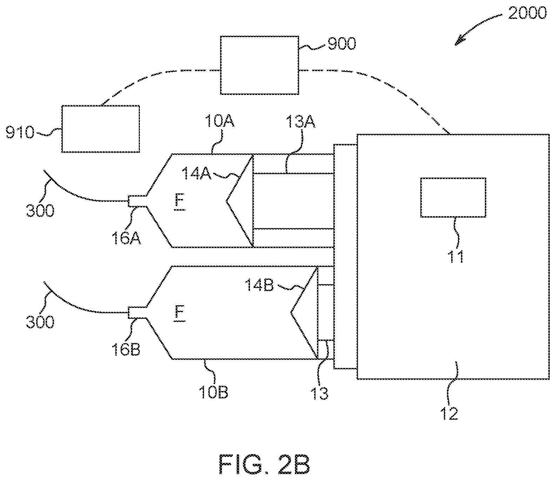

Referring now to **FIG. 2B****,** the fluid injector system **2000** is shown with a prime tube **300** attached to each of the syringes **10A, 10B.** The prime tubes **300** may be attached to the syringes **10A, 10B** after a filling operation in which the syringes **10A, 10B** are filled from the bulk fluid containers **19A** and **19B** and prior to an injection procedure. Other embodiments may be directed towards priming of at least a portion of a tubing set in preparation for a fluid injection procedure. For example, in one embodiment, the prime tube configurations described herein may be attached to a distal end of a tubing set (as shown in **FIG. 2A**), such as a multi-use portion of a tubing set and used to ensure priming of air from the syringe and the multi-use portion of the tubing set. In other embodiments, the prime tube may be attached to a distal end of a newly installed single patient tubing set (attached to the multi-use tube set) to ensure priming of the single patient tubing set prior to attaching to a patient catheter. The fluid paths **210A, 210B** may be disconnected from the syringes **10A, 10B** to allow for connection of the prime tubes **300.** With the prime tubes **300** connected to the syringes **10A, 10B,** the fluid injector system **2000** may be actuated to prime/purge the syringes **10A, 10B** by extending the pistons **13A, 13B** distally to eject air from the syringes **10A, 10B** into the associated prime tubes **300.** In some embodiments, the controller **900** may be programmed or configured to extend each of the pistons **13A, 13B** a predetermined distance, corresponding to a predetermined fluid volume, to eject the predetermined air/fluid volume from each syringe **10A, 10B** into the associated prime tubes **300.** The predetermined air/fluid volume may be selected to ensure that all (or substantially all) of the air in the syringes **10A, 10B** is ejected into the prime tubes **300.** For example, the predetermined air/fluid volume may be selected based on empirical data of the maximum volume of air within syringes after a fill operation. The predetermined air/fluid volume may include a factor of safety to ensure that even an abnormally high amount of air will be fully ejected from the syringe during the priming/purging operation. As the predetermined air/fluid volume is typically larger than the actual volume of air present in the syringes **10A, 10B,** some amount of the medical fluid **F** will be ejected into the prime tubes **300** along with the volume of air. Once the priming operation is complete, the prime tubes **300** may be disconnected from the syringes **10A, 10B** and the fluid paths **210A, 210B** may be reconnected in the arrangement shown in **FIG. 2A** in preparation for an injection procedure. The prime tubes **300** may be configured to retain the medical fluid **F** after the prime tubes **300** are disconnected from the syringes **10A, 10B** to prevent spills and leaks. In some embodiments, prime tubes **300** may be discarded once disconnected from syringes **10A, 10B.**

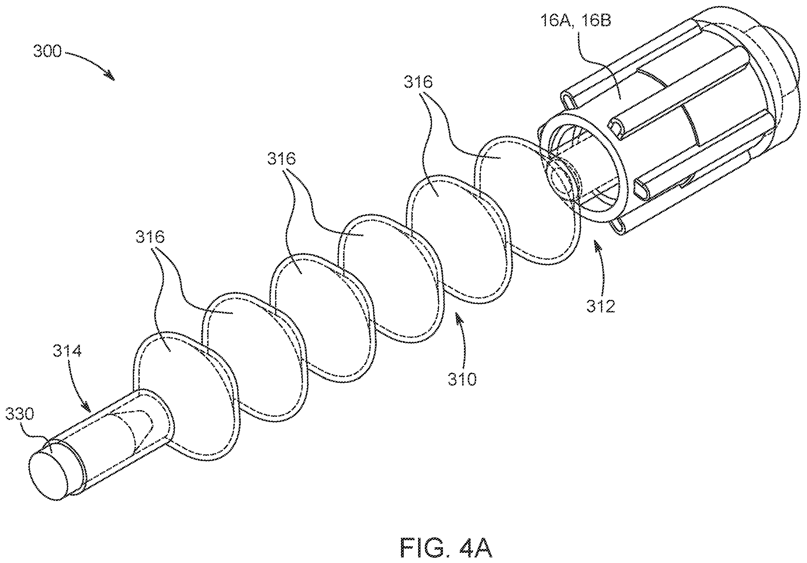

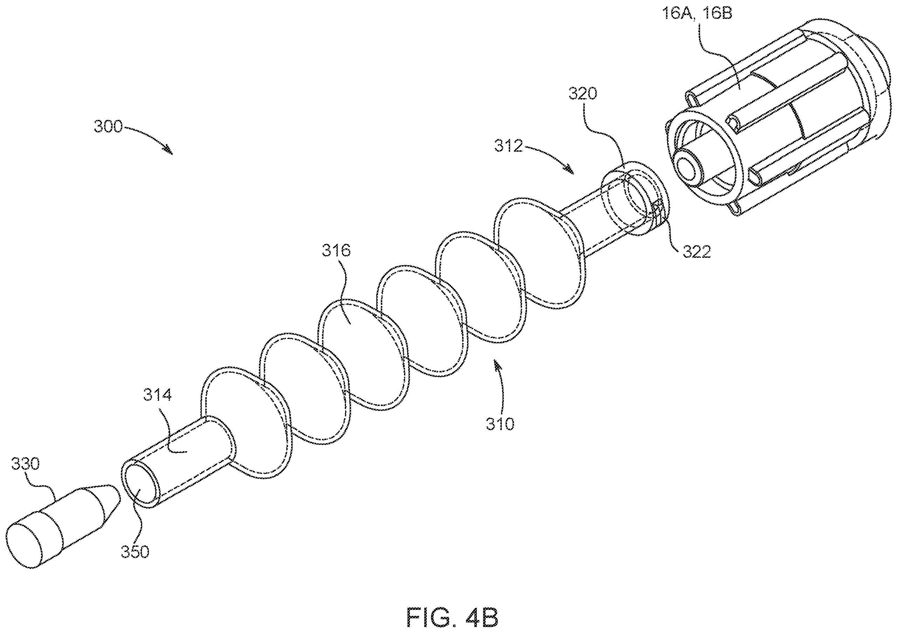

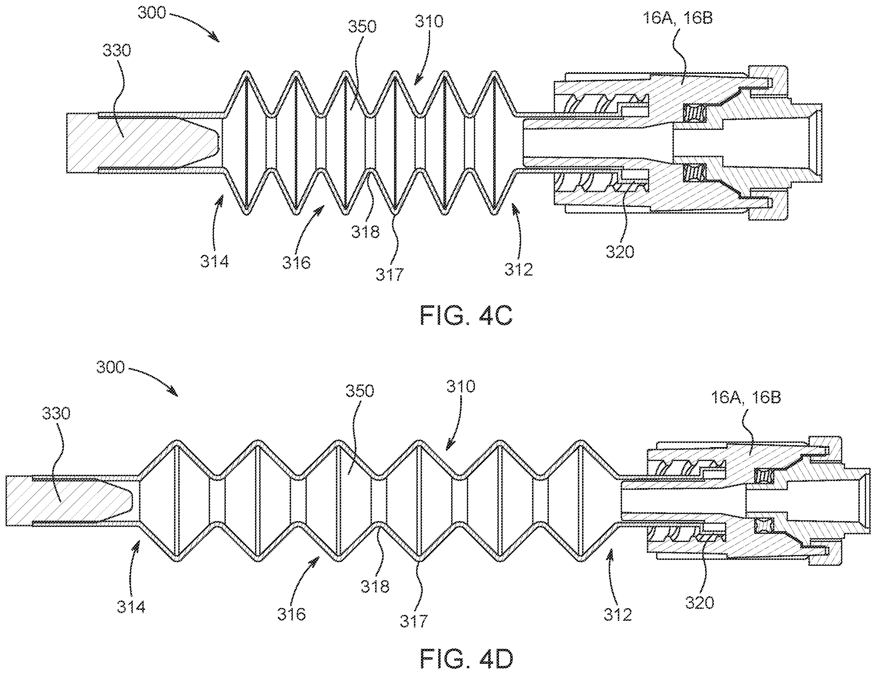

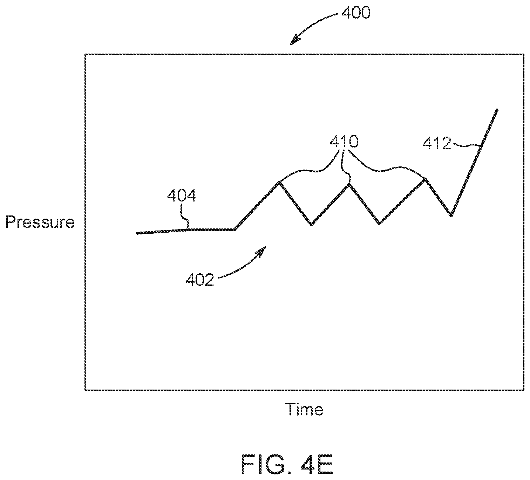

In some embodiments, the fluid injector system **2000** may include one or more priming status sensors **910** associated with the prime tubes **300** and/or the syringes **10A, 10B.** The priming status sensors **910** may be in communication with the controller **900** and may be configured to detect a priming status, e.g. whether the syringes **10A, 10B** have been primed to remove all air therefrom, based on observation and/or measurement of air and fluid in the prime tubes **300.** In various embodiments, the priming status sensors **910** may include optical sensors (e.g. cameras), strain gauges, microphones, ammeters, limit switches, pressure transducers, or any other variety of sensor capable of detecting a property of the system **2000** that can be used to determine the priming status. Further details of the priming status sensors **910** will be provided in the context of particular embodiments of the prime tubes **300** as described herein with reference to **FIGS. 4-15B****.**

Referring now to **FIG. 3****,** in some embodiments, the prime tube **300** may include a sidewall **310** having a proximal end **312** and a distal end **314** and defining an internal chamber **350.** The proximal end **312** of the sidewall **310** may include a connector **320** configured to reversibly engage the outlet **16A, 16B** of the associated fluid reservoir, such as syringe **10A, 10B.** In some embodiments, the connector **320** may include one or more engaging lugs **322** having a profile complementary to an inner threading of the outlet **16A, 16B** of the syringe **10A, 10B.** In some embodiments, the one or more engaging lugs **322** may be configured to release from the outlet **16A, 16B** of the syringe **10A, 10B** at a predetermined fluid pressure within the prime tube **300.** In some embodiments, the connector **320** may be a female luer connector. In other embodiments, the connector may be a male or female portion of the connector and configured to interface/connect with the corresponding complementary female or male connector as described in International PCT Application No. PCT/US2021/018523.

In some embodiments, the prime tube **300** may include a closure **330** associated with the distal end **314** of the sidewall **310.** The closure **330** may be permeable to air and substantially impermeable to liquids, including the medical fluid **F.** For example, according to various embodiments, the closure **330** may be a porous material, such as a material having at least one aperture having a cross-sectional area sized to allow to allow passage of air and substantially prohibit passage of a medical fluid through the at least one aperture. As such, during a priming operation of the syringes **10A, 10B,** the air ejected from the syringes **10A, 10B** by pressurization of the syringe contents may flow out of the prime tube **300** via the closure **330,** while any medical fluid **F** ejected from the syringe **10A, 10B** is retained within the internal chamber **350.** Due to the differences in compressibility of a gas, such as air, and a liquid, such as the medical fluid **F** (e.g., contrast or saline) pressure build-up in the prime tube **300** substantially increases after all the air has been ejected from the syringe and liquid is ejected into the prime tube **300.** According to various embodiments, the pressure differential between the air and the liquid medical fluid may be used to determine the prime status of the syringe. In some embodiments, the closure **330** may be made from a hydrophobic medical grade plastic, such as those commercially available under the trade names Gore-tex^{®} and Porex^{®}, which generally allow passage of gas but not liquid therethrough. In some embodiments, the closure **330** may be a solid material defining one or more apertures having a sufficient cross-sectional area to allow the passage of air out of the prime tube **300,** but an insufficient cross-sectional area to allow the passage of the medical fluid **F.** In some embodiments, the closure **330** may include an absorbent material, such as cotton or other fibrous material, to absorb the medical fluid **F** while allowing air to pass out of the prime tube **300.** In some embodiments, the closure **330** may be a solid member impermeable to all fluids, e.g. air and medical fluids, such that all fluids injected into the prime tube **300** from the syringe **10A, 10B** are retained within the internal chamber **350.**

In some embodiments, the closure **330** may include a crack pressure valve, such as a high crack pressure valve, configured to open in response to a predetermined fluid pressure. A such, the closure **330** may remain closed as pressure builds within the prime tube **300,** and the closure **330** may open at the predetermined fluid pressure to allow air and/or fluid out of the prime tube **300.**

In some embodiments, the internal chamber **350** may define an expandable volume. That is, the volume of the internal chamber **350** may increase as the sidewall **310** stretches, unrolls, unfolds, or otherwise changes shape. In such embodiments, the sidewall **310** may be made from a stretchable, foldable, and/or resilient material. In some embodiments, the expandable volume of the internal chamber **350** increases as fluid enters the internal chamber **350** such that the internal chamber **350** adopts the volume of the fluid ejected from the syringe **10A, 10B.** In some embodiments, the internal chamber **350** may be configured to expand or become rigid in response to an increase in fluid pressure as fluid is ejected into the internal chamber **350.**

In some embodiments, the sidewall **310** may be biased toward a contracted state, e.g. a state in which the prime tube **300** is supplied and initially connected to the syringe **10A, 10B** wherein the internal chamber **350** has a minimum volume. According to certain configurations, once the internal chamber **350** expands due to an increase in fluid pressure, such fluid pressure must be maintained or the internal chamber **350** will have a tendency to return, at least partially, to the contracted state and eject the fluid back out of the connector **320.** According to these configurations, a one-way valve **340,** such as a check valve, may be disposed adjacent to or integral with the connector **320** of the prime tube **300** to prevent fluid flow out of the prime tube **300** when fluid pressure is relieved, e.g. when the prime tube **300** is disconnected from the syringe **10A, 10B.** Thus, inadvertent spills or leakage of the medical fluid when the prime tube **300** is removed from the syringe are avoided.

In some embodiments, the sidewall **310** may be stable in both the contracted state and the expanded state, in that the internal chamber **350** is not biased toward either the contracted state or an expanded state. In such embodiments, relief of fluid pressure from the internal chamber **350,** such as when the prime tube **300** is disconnected from the syringe **10A, 10B,** does not result in the internal chamber **350** returning to the contracted state. As such, the one-way valve **340** may not be required to prevent fluid flow back out of the prime tube **30.** However, the one-way valve **340** may still be provided to prevent inadvertent discharge of fluid, such as if the technologist squeezes the sidewall **310** during removal of the prime tube **300** from the syringe **10A, 10B** or accidently tips the prime tube **300** so that gravity causes the fluid therein to flow through the connector **320.**

The shape change of the sidewall **310** due to the expansion of the internal chamber **350** may be used as an indicator that the priming/purging operation of the syringe **10A, 10B** has been completed. The shape change of the sidewall **310** may be detected by priming status sensor **910,** and the priming status sensor **910** may send an output signal to controller **900** indicating the priming status of syringes **10A, 10B.** In other embodiments, the technician may visually or audibly determine that a priming operation is completed.

In some embodiments, the sidewall **310** may be substantially rigid, meaning that the sidewall does not substantially deform under the pressure associated with an injection procedure, e.g. 1200 psi for a CV procedure.

In some embodiments, the internal chamber **350** may have a volume sufficient to hold the predetermined fluid volume ejected from the associated syringe **10A, 10B** during the syringe priming/purging operation. In some embodiments, the volume defined by the internal chamber **350** may range from 5 mL to 30 mL and in other embodiments, from 5 mL to 10 mL. In specific embodiments, the volume should be sufficient to account for cases when more than one prime sequence is required, for example when air bubbles are still observed in the system. In certain embodiments, a prime operation may include two injection sequences, a first forceful flow rate with small ejected volumes to remove the majority of air from system and then a second slower flow rate with larger flow volumes, optionally accompanied by tapping or vibrations, to remove small air bubbles.

To perform a priming/purging operation to expel air from the syringe **10A, 10B,** the syringe **10A, 10B** may be held in a substantially upright vertical position with the outlet **16A, 16B** at the highest point, the syringe **10A, 10B** may be primed, e.g., the pistons **13A, 13B** moved in the distal direction to expel a portion of the contents of the syringe (i.e., air and medical fluid **F**), to purge any air from the interior of the syringe **10A, 10B.** In the vertical position, a majority of the air in the syringe **10A, 10B** rises to the distal end of the syringe adjacent the outlet **16A, 16B** due to buoyancy of air in the medical fluid **F.** As the syringe **10A, 10B** is primed, the air at the distal end of the syringe **10A, 10B** is expelled through the outlet **16A, 16B** and enters the internal chamber **350** of the prime tube **300.** During the priming operation, air may be dislodged from the various interior surfaces of the syringe and the surface of the plunger to ensure that all air is primed. Examples of such methods for dislodging air bubbles (including microbubbles) from an interior surface or plunger surface are described in International PCT Application Publication No. WO 2019/204617, for example by placing the syringe contents under an at least partial vacuum to coalesce the small air bubbles into larger bubbles and/or vibrating, knocking, or otherwise impacting the syringe or piston/plunger assembly to dislodge the air bubbles.

Referring now to **FIGS. 4A-15B****,** various embodiments of the prime tube **300** are illustrated. Components in the embodiments shown in **FIGS. 4A-15B** having the same reference numerals as the embodiment of **FIG.3** indicate similar and/or functionally equivalent components. Referring first to **FIGS. 4A-4D****,** an embodiment of a prime tube **300** includes an expanding sidewall **310** having at least one bellows **316.** The at least one bellows **316** are configured to expand and contract longitudinally to increase the volume of the internal chamber extending longitudinally between the proximal end **312** and the distal end **314** of the sidewall **310.** The at least one bellows **316** include alternating wider diameter sections **317** and narrow diameter sections **318** (see **FIG. 4D**). Initially, the at least one bellows **316** is provided in a contracted state (see **FIG. 4C**) in which the distance between the proximal-most wider diameter section **317** and the distal-most wider diameter section **317** is minimized. In the contracted state of **FIG. 4C****,** the length of the sidewall **310** shorter than in the expanded state shown in **FIG. 4D** and the interior volume of the internal chamber **350** is minimized. Consequently, the volume defined by the internal chamber **350** is greater in the expanded state.

With continued reference to **FIGS. 4A-4D****,** the closure **330,** in some embodiments in the form of a porous plug formed of a porous hydrophobic polymer or other material as described herein and is substantially permeable to air and impermeable to liquid, is inserted or otherwise connected to the distal end **314** of the prime tube **300.** The closure **330** may be configured to allow air or other gas to pass through the pores of the material while preventing a medical fluid **F** from exiting the internal chamber **350.** It should be recognized that the position of the porous plug may be located elsewhere on the prime tube **300** while still allowing air to escape the prime tube **300** and retain liquid in the prime tube **300.**

During a priming/purging operation of the syringe **10A, 10B,** air ejected from the syringe **10A, 10B** passes through the internal chamber **350** and exits through the closure **330** at the distal end **314.** Due to the compressibility of air, the configuration of the at least one bellows **316** remains substantially unchanged and in the contracted state. As the air is continued to be primed, eventually some medical fluid **F** pushes the remaining air from the syringe **10A, 10B** into the prime tube **300** and some amount of medical fluid **F** itself enters the prime tube **300.** The medical fluid **F** moves through the at least one bellows **316** and contacts the porous plug of the closure **330,** through which the medical fluid **F** is unable to pass. Consequently, fluid pressure builds in the internal chamber **350** and the at least one bellows **316** begin to expand from the compressed state to the extended state (compare **FIG. 4C to FIG. 4B**).

Expansion of the bellows **316** of the sidewall **310** may be visualized by the technologist to confirm that substantially all air has been purged from the syringe **10A, 10B** and that the syringe **10A, 10B** is primed and ready for use. In some embodiments, the priming status sensor **910** (see **FIGS. 2B** and **3**) may include a detector for image recognition, such as a camera as described in U.S. Patent No. 10,201,666. Based on an output signal from the priming status sensor **910,** the controller **900** may be programmed or configured to determine that the prime tube **300** is in the expanded state and the syringe **10A, 10B** is primed of air. The processor **900** may be configured to prevent a fluid injection procedure until it receives indication that the syringe **10A, 10B** has been successfully primed. Once a successful prime has been established as determined by the controller **900** and/or the technologist, the prime tube **300** may be removed and discarded according to hospital protocol, and a suitably primed patient catheter line may be installed on the syringe **10A, 10B.** It should be noted that while the prime tube embodiments described herein are utilized to ensure priming of air from a syringe, the various embodiments of the prime tube may also be used to ensure priming of air from a tubing set as well as the syringe. According to these embodiments, the prime tube may be placed at the distal end of the tubing set (as shown in **FIG. 2A**) and used in a similar manner to determine with all air has been effectively ejected from the internal volume of the tubing set, and in certain embodiments, form the corresponding syringe(s) and/or peristaltic pump(s) mechanism attached to the proximal end(s) of the tubing set.

With continued reference to **FIGS. 4A-4D****,** in some embodiments, each of the bellows **316** may be configured to emit an audible sound, such as a pop, when transitioning from the contracted state to the expanded state. The technologist may use the sound as an indication that substantially all air has been purged from the syringe **10A, 10B** and the syringe **10A, 10B** is primed and ready for use. Additionally, the priming status sensor **910** may include a microphone configured to detect the sound and send a signal to the processor **900,** which may in turn determine that the syringe **10A, 10B** has been primed based on the signal. For example, the technologist or processor **900** may count the number of pops corresponding to the expansion of the at least one bellows **312** and relate that to the total number of bellow features to determine when the prime tube **300** is in the completely expanded configuration.

Referring now to **FIG. 4E****,** a graph **400** illustrating fluid pressure within the prime tube **300** against time is shown for the embodiment of **FIGS. 4A-4D****.** The fluid pressure, represented by curve **402,** is initially substantially constant at section **404** as primarily air is injected into the internal chamber **350** and flows through the porous material of the closure **330.** The fluid pressure then experiences several peaks **410** as medical fluid **F** enters the internal chamber **350** and causes each of the at least one bellows **316** to transition from the contracted state to the expanded state. In particular, because the medical fluid **F** cannot flow out of the distal end **314,** the fluid pressure builds up until a first of the at least one bellows **316** transitions to the expanded state. As the first of the at least one bellows **316** expands, the fluid pressure drops due to the increase in volume of internal chamber **350** resulting from expansion of the bellows **316.** As more fluid is expelled from the syringe **10A, 10B** into the internal chamber **350,** the pressure again builds until another of the bellows **316** expands. This process continues until all of the bellows **316** are in the expanded state and the priming operation is completed. After all bellows **316** are in the expanded state, the fluid pressure will then rise, as indicated by the tail section **412** of the curve **402,** until the fluid flow is stopped.

In some embodiments, the priming status sensor **910** may include a motor current (or motor force) sensor configured to measure the current draw of the drive motor associated with the piston **13A, 13B.** The controller **900** may be programmed or configured to detect changes in the fluid pressure based on changes in motor current. In particular, changes in motor current may correlate to changes in fluid pressure within the prime tube **300,** as shown in the graph of **FIG. 4E****.** When the motor current, and thus the fluid pressure continues to rise after the last bellows **316** is in the expanded state, the controller **900** may then stop the priming operation and indicate that the system is primed and can proceed to the next step in the injection protocol. Alternatively, after a predetermined number of bellows **316** have expanded, the controller **900** may determine that the system has been effectively primed.

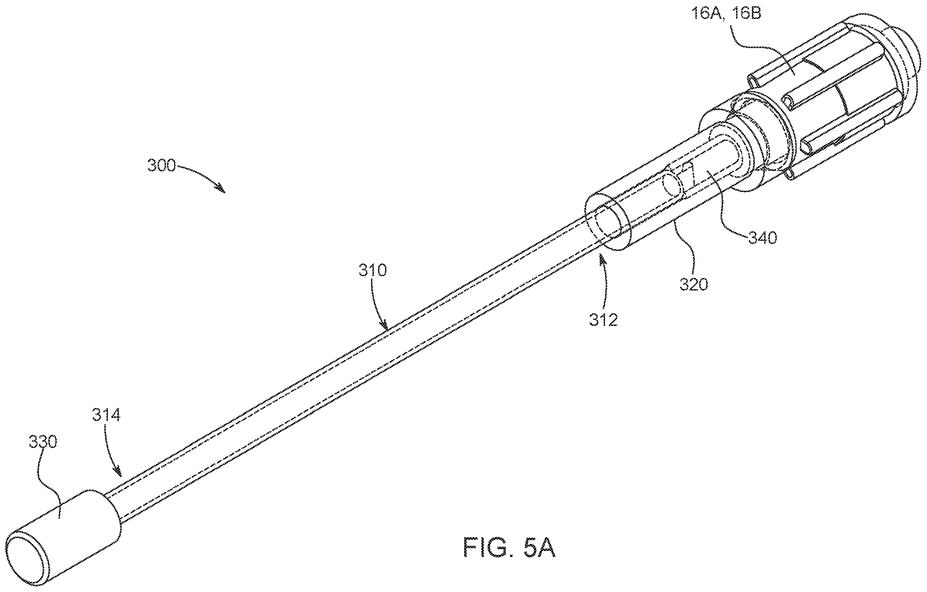

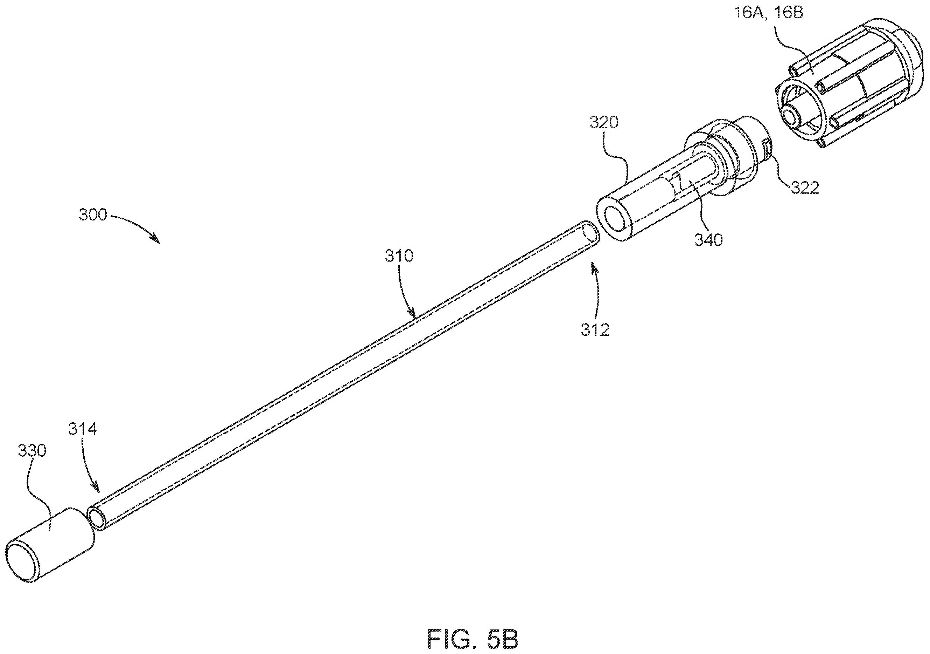

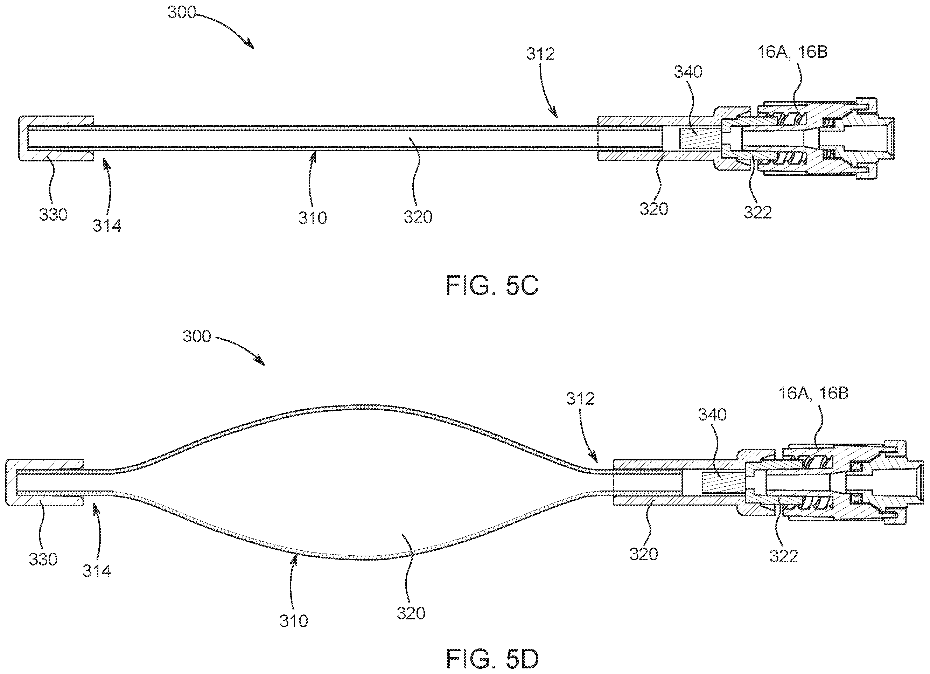

Referring now to **FIGS. 5A-5D****,** an embodiment of a prime tube **300** including an expandable sidewall **310** is illustrated. The sidewall **310** may be made of a flexible or stretchable elastomeric material or balloon which expands when there is a pressure differential between the internal chamber **350** and the environment outside of the sidewall **310.** The material of the sidewall **310** may be elastic in that when stretched, the sidewall **310** attempts to return to its initial state. Thus, if fluid pressure is removed from the internal chamber **350,** the sidewall **310** will contract and force fluid back out of the proximal end **312** of the prime tube **300.** The one-way valve **340** is provided to prevent backflow out of the proximal end **312** when the prime tube **300** is disconnected from the syringe. The prime tube **300** is provided in the non-expanded configuration shown in **FIGS. 5A-5C****.**

With continued reference to **FIGS. 5A-5D****,** the closure **330** in one embodiment may be a sealing plug inserted or otherwise connected to the distal end **314** of the prime tube **300.** The sealing plug may prevent all fluid, including both air and medical fluid **F,** from exiting the distal end **314** of the prime tube **300.** Due to the sealed nature of the sidewall **310** because of the one-way valve **340** and the closure **330,** air and liquid is trapped in the internal chamber **350** during the priming operation. As the air is continued to be primed, eventually some medical fluid **F** pushes the remaining air into the prime tube **300** and the medical fluid **F** itself enters the internal chamber **350.** As the volume of air and subsequently the fluid in the sidewall **310** increases, the pressure in the sidewall **310** increases and the flexible expandable walls of the sidewall **310** expand, similar to inflation of a balloon. In particular, as the pressure builds in the prime tube **300,** the sidewall **310** begins to expand from the compressed state shown in **FIGS. 5A-5C** to the expanded state shown in **FIG. 5D****.** Once the volume of the internal chamber **350** reaches a specific size, the technologist may determine that the syringe **10A, 10B** has been primed and is ready for an injection procedure. In certain embodiments, the elastic characteristics of the sidewall **310** may be chosen so that it does not substantially expand under the pressurized air - due to the compressibility of gases - but expands when liquid pressure builds in the internal chamber **350.**

In other embodiments, the closure **330** may be porous such that air can pass therethrough but it is impermeable to liquid. According to this embodiment and similar to the bellows (**FIG. 4A-4E**), the elastomeric sidewall **310** does not substantially expand when air flows into the internal chamber **350** since such air flows out through the closure **330.** However, when liquid medical fluid **F** is injected into the internal chamber **350,** the elastomeric sidewall **310** expands and the fluid pressure builds therein.

In some embodiments the priming status sensor **910** may include a camera, as described herein in connection with **FIGS. 3** and **4A****-4D,** in conjunction with the controller **900** to determine that the syringe **10A, 10B** has been primed based on the sidewall **310** being in the expanded state. As described herein in connection with **FIGS. 4A-4D****,** the controller **900** may be programmed or configured to prevent performance of an injection procedure until a successful prime of the syringe **10A, 10B** has been detected]

During removal of the prime tube **300** from the syringe **10A, 10B,** the one-way valve **340** prevents the pressurized air and/or fluid in the internal chamber **350** from releasing from the proximal end **312** of the prime tube **300.** The entire prime tube **300** with air and medical fluid **F** contained therein may be discarded according to hospital protocol.

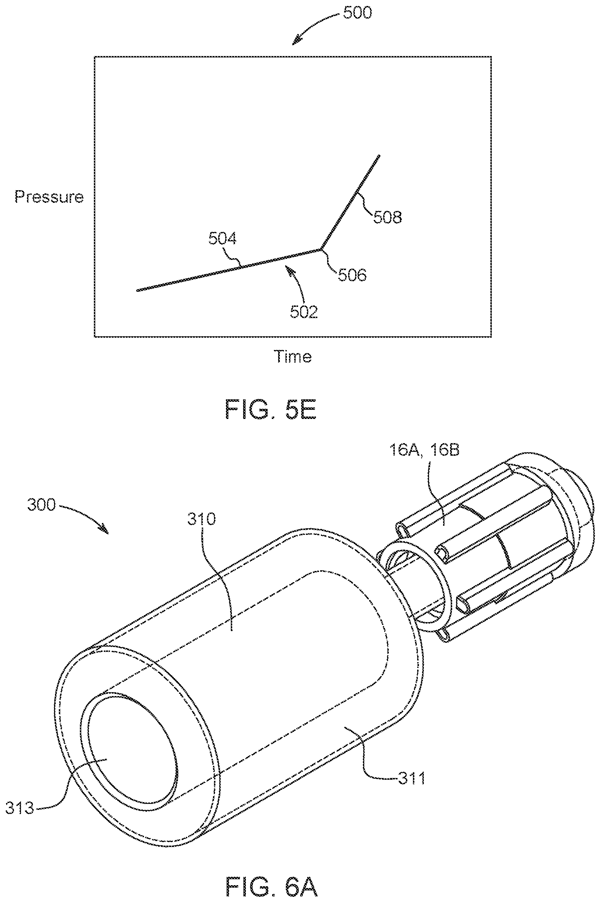

Referring now to **FIG. 5E****,** a graph **500** illustrating fluid pressure within the prime tube **300** against time is shown for the prime tube of **FIGS. 5A-5D****.** The fluid pressure, represented by curve **502,** initially rises slowly at section **504** as primarily air is injected into the internal chamber **350** with a low slope due to the compressibility of air. The fluid pressure continues to rise at a greater rate as medical fluid **F** pushes the remaining air out of the syringe **10A, 10B** and some medical fluid **F** itself enters the internal chamber **350.** The fluid pressure may experience a transition point **506** at which the sidewall **310** experiences a yield due to expansion of the sidewall **310.** Once the total expanded volume of the internal chamber **350** is neared, the pressure increases at an even greater rate at section **508.** In some embodiments, the priming status sensor **910** may include a motor current (or motor force) sensor configured to measure the current draw of the drive motor associated with the piston **13A, 13B.** The controller **900** may be programmed or configured to detect changes in the fluid pressure based on changes in motor current and relate the pressure measurement to prime status. In particular, changes in motor current may correlate to changes in fluid pressure within the prime tube **300,** as shown in the graph of **FIG. 5E** allowing controller **900** to determine when the system has been effectively primed.

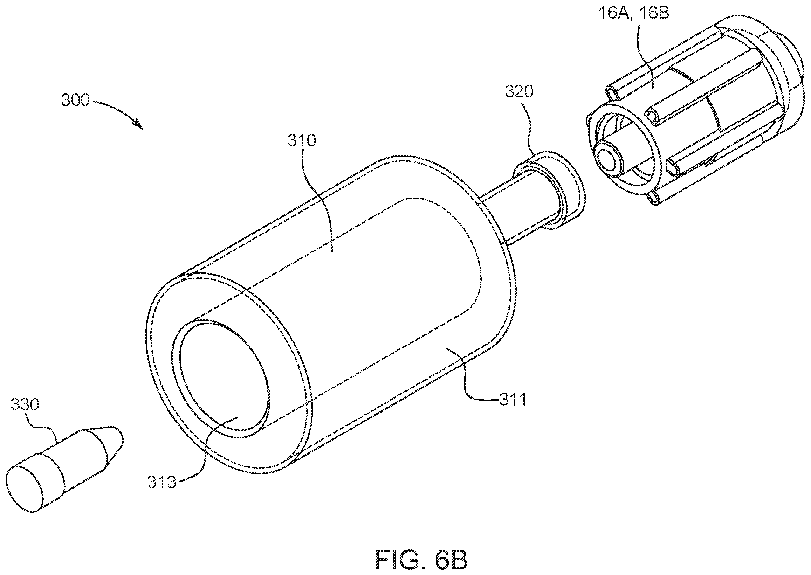

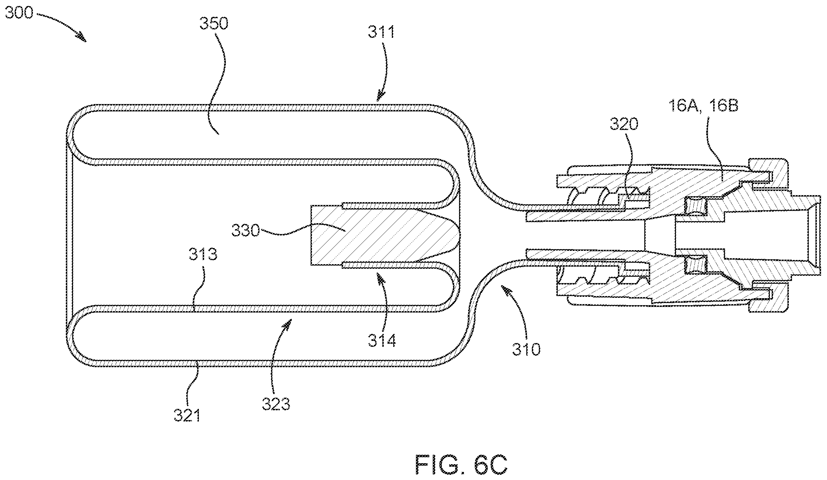

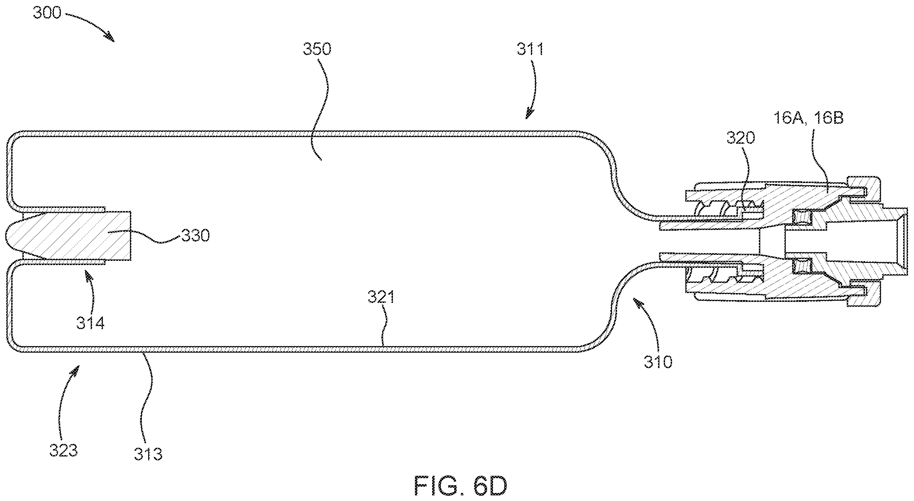

Referring now to **FIGS. 6A-6D****,** an embodiment of a prime tube **300** is illustrated in which the sidewall **310** includes an expanding rolling diaphragm body. The sidewall **310** is flexible or resilient such that the sidewall **310** may be rolled or folded over itself in response to fluid pressure in the internal chamber **350.** In the contracted state in which the prime tube **300** is initially provided, shown in **FIGS. 6A-6C****,** the sidewall **310** is arranged such that a distal portion **313** of the sidewall **310** is rolled or folded into an interior space defined by a proximal portion **311** of the sidewall **310.** The distal portion **313** is rolled over the proximal portion **311** such that an inner surface **323** of the distal portion **313** faces an inner surface **321** of the proximal portion **311.** At least a portion of the distal portion **313** of the sidewall **310** is configured to invert in response to an increase in fluid pressure within the internal chamber **350** to transition the sidewall **310** to the expanded position shown in **FIG. 6D****.** In particular, the distal portion **313** inverts by unrolling/unfolding from inside the proximal portion **311,** such that the sidewall **310** assumes the expanded state shown in **FIG. 6D****.** Consequently, the volume defined by the internal chamber **350** is greater with the sidewall **310** in the expanded state than in the contracted state.

With continued reference to **FIGS. 6A-6D****,** the closure **330** in this embodiment may include a porous material substantially the same as the embodiment of **FIGS. 4A-4D** to allow air to flow through the closure **330** while retaining medical fluid **F** in the internal chamber **350.** During a priming/purging operation of the syringe, air injected into the prime tube **300** from the syringe **10A, 10B** passes through the internal chamber **350** and exits through the porous material of the closure **330** at the distal end **314.** Due to the compressibility of air, the rolling diaphragm portion of the sidewall **310** remains substantially unchanged, i.e., remains in the contracted state, as air is ejected from the syringe **10A, 10B.** As the air is continued to be primed, the medical fluid **F** pushes the remaining air from the syringe **10A, 10B** into the prime tube **300** and out the porous material of the closure **330,** and the medical fluid **F** itself enters the internal chamber **350.** Due to the hydrophobic nature of the closure **330,** the medical fluid **F** is unable to exit the internal chamber **350.** As the volume of the medical fluid **F** in the internal chamber **350** increases, the pressure in the internal chamber **350** increases, forcing the distal portion **313** to unroll/unfold from the interior of the proximal portion **311,** thus transitioning the sidewall **310** from the contracted state shown in **FIGS. 6A-6C** to the expanded state shown in **FIG. 6D****.**

Once the volume of the internal chamber **350** reaches a specific size or the rolling diaphragm portion of the sidewall **310** reaches a specific unrolled state, e.g., completely unrolled, the technologist may determine that substantially all air has been purged from the syringe **10A, 10B,** meaning the syringe **10A, 10B** is primed and ready for use. In some embodiments, the priming status sensor **910** (see **FIGS. 2B** and **3**) may include a detector for image recognition, such as a camera as described in U.S. Patent No. 10,201,666. Based on an output signal from the priming status sensor **910,** the controller **900** may be programmed or configured determine that the prime tube **300** is in the expanded state and the syringe **10A, 10B** is primed of air. The processor **900** may be configured to prevent a fluid injection procedure until it receives indication that the syringe **10A, 10B** has been successfully primed. Once a successful prime has been established as determined by the controller **900** and/or technologist, the prime tube **300** may be removed and discarded according to hospital protocol, and a suitably primed patient catheter line may be installed on the syringe **10A, 10B.**

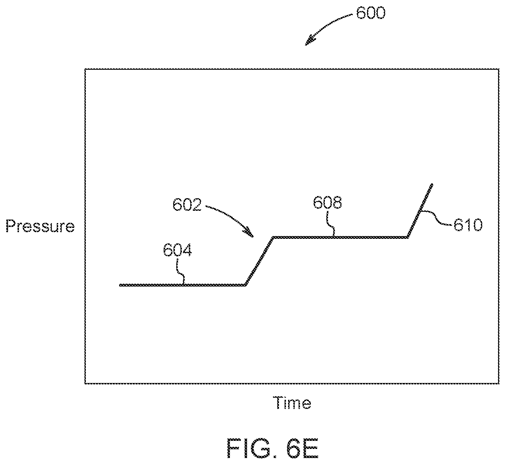

Referring now to **FIG. 6E****,** a graph **600** illustrating fluid pressure within the prime tube **300** against time is shown for the embodiment of **FIGS. 6A-6D****.** The fluid pressure, represented by curve **602,** is initially substantially constant at section **604** as primarily air is injected into the internal chamber **350** and flows through the porous material of the closure **330.** The fluid pressure then increases as medical fluid **F** enters the internal chamber **350,** until the fluid pressure reaches a sufficient pressure to cause the distal portion **313** of the sidewall **310** to unroll from the proximal portion **311.** The fluid pressure then continues to remain substantially constant at section **608** as more fluid is expelled from the syringe **10A, 10B** into the internal chamber **350,** and continues to cause the sidewall **310** to unroll towards the expanded state. After the sidewall **310** is in the substantially unrolled state the volume of the internal chamber **350** is substantially maximized, the fluid pressure rises substantially at section **610** with continued ejection of fluid from the syringe.

In some embodiments, the priming status sensor **910** may include a motor current (or motor force) sensor configured to measure the current draw of the drive motor associated with the piston **13A, 13B.** The controller **900** may be programmed or configured to detect changes in the fluid pressure based on changes in motor current. In particular, changes in motor current may correlate to changes in fluid pressure within the prime tube **300,** as shown in the graph of **FIG. 6E****.**

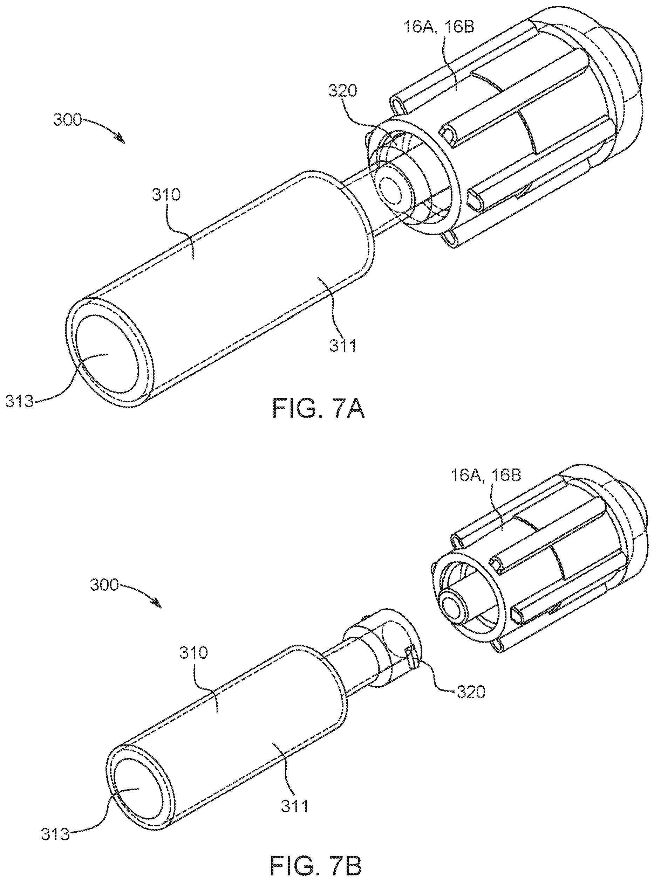

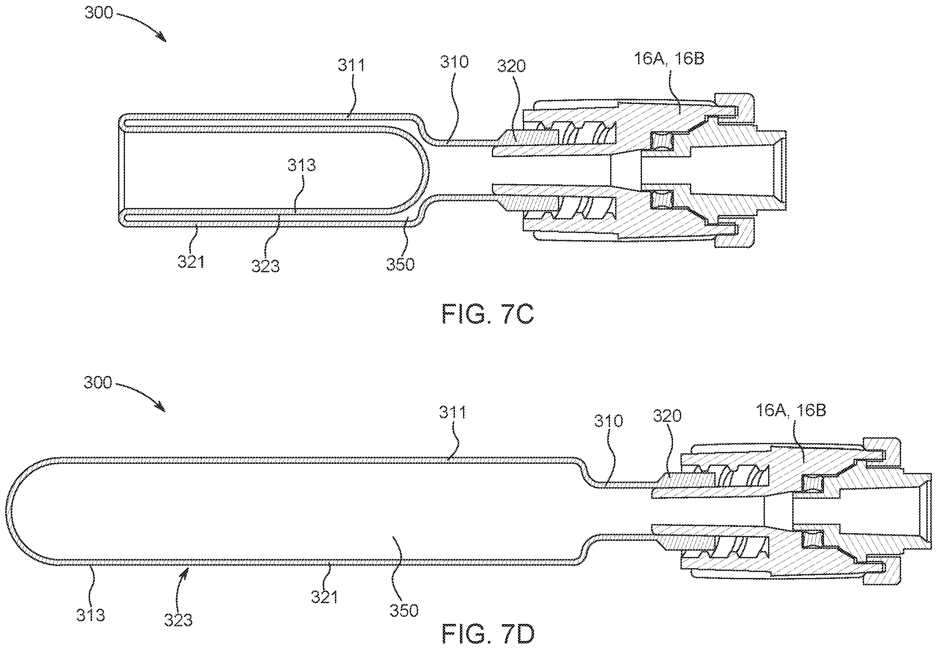

Referring now to **FIGS. 7A-7D****,** an embodiment of a prime tube **300** is illustrated which includes many of the same features, such as the rolling diaphragm sidewall **310,** of the embodiment of **FIGS. 6A-6D****.** The primary difference between these embodiments is that the distal end **314** of the sidewall **310** is completely sealed in the embodiment of **FIGS. 7A-7D****,** such that air cannot exit the distal end **314.** As a result, all fluid, including both air and medical fluid **F,** ejected from the syringe **10A, 10B** during the priming/purging operation is retained within the internal chamber **350.** Due to the compressibility of air and non-compressibility of the medical fluid, the rolling diaphragm will remain substantially in the rolled state as air is ejected into prime tube **300** but then transition to the expanded state as fluid is ejected into the internal chamber **350** and the fluid pressure within the internal chamber **350** builds. Prime states may then be recognized by wither the technologist or by the controller **900** using input from a priming status sensor **910,** as described herein.

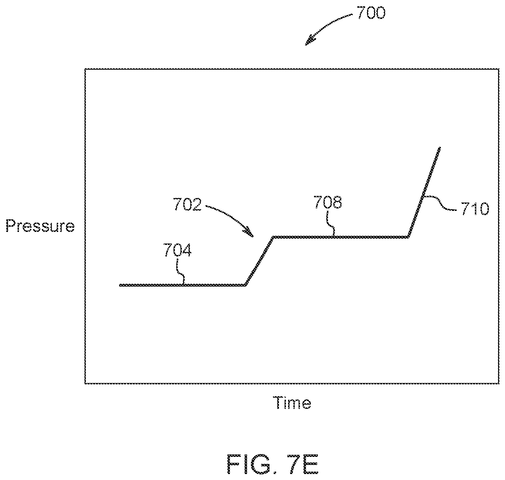

Referring now to **FIG. 7E****,** a graph **700** illustrating fluid pressure within the prime tube **300** against time is shown for the embodiment of **FIGS. 7A-7D****.** The fluid pressure, represented by curve **702,** is initially substantially constant at section **604** as primarily air is injected into the internal chamber **350** and flows through the porous material of the closure **330.** The fluid pressure then increases as medical fluid **F** enters the internal chamber **350,** until the fluid pressure reaches a sufficient pressure to cause the distal portion **313** of the sidewall **310** to unroll from the proximal portion **311.** The fluid pressure then continues to remain substantially constant at section **708** as more fluid is expelled from the syringe **10A, 10B** into the internal chamber **350,** and continues to cause the sidewall **310** to unroll towards the expanded state. After the sidewall **310** is in the substantially unrolled state the volume of the internal chamber **350** is substantially maximized, the air/fluid pressure rises substantially at section **710** with continued ejection of fluid from the syringe **10A, 10B.** The pressure behavior of the embodiment of **FIGS. 7A-7D** is thus similar to the embodiment of **FIGS. 6A-6D****,** although the presence of the porous closure **330** in the embodiment of **FIGS. 6A-6D** may at least initially reduce pressure within the internal chamber **350** as air is able to escape.

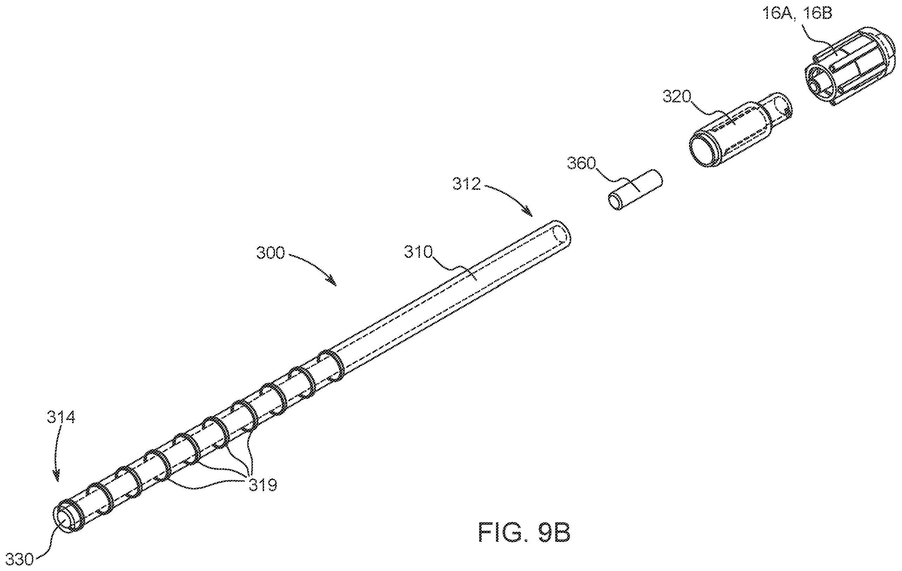

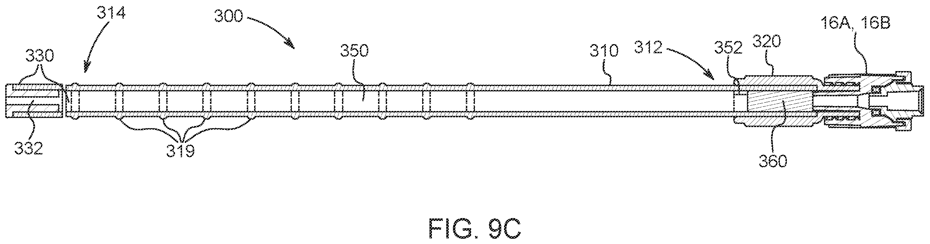

Referring to **FIGS. 8A-9C****,** embodiments, not according to the claimed invention, of a prime tube **300** including a shuttle member **360** slidable within the internal chamber **530** is illustrated. According to these embodiments, the sidewall **310** may be substantially rigid and non-elastic, such that under normal priming pressures below a predetermined threshold the sidewall **310** does not appreciably expand. The shuttle member **360** is configured to slide within the internal chamber **350** from the proximal end **312** toward the distal end **314** in response to liquid being ejected into the prime tube **300** from the syringe **10A, 10B.** The prime tube **300** is initially provided with the shuttle member **360** adjacent to or near the proximal end **312.** In some embodiments, the shuttle member **360** may be sized to have a frictional fit with the sidewall **310** such that the shuttle member **360** cannot be inadvertently dislodged from the proximal end **312** until the prime tube **300** is pressurized from the syringe **10A, 10B.** In some embodiments, the proximal end **312** of the sidewall **310** has a slightly reduced diameter to increase the frictional fit with the shuttle member **360** at the proximal end **312.** As such, the initial amount of fluid pressure required to dislodge the shuttle member **360** is greater than the fluid pressure required to move the shuttle member **360** the remainder of the distance to the distal end **314.** In some embodiments, as shown in **FIG. 9C****,** the sidewall **310** may include an internal lip **352** to retain the shuttle member **360** adjacent to or near the proximal end **312** until the prime tube **300** is pressurized from the syringe **10A, 10B.** The lip **352** or friction fit may be such that the shuttle member **360** is not dislodged under air pressure but is dislodged and becomes slidable when fluid flows into the internal chamber **350.**

In some embodiments, the shuttle member **360** may allow passage of air to the distal end **314** of the prime tube **300.** In such embodiments, the shuttle member **360** may be made of a hydrophobic medical grade plastic, as described herein, that are permeable to air but impermeable to the medical fluid **F.** In some embodiments, the dimensional tolerance between the outside of the shuttle member **360** and an the inside of the sidewall **310** may be such that air can pass between the shuttle member **360** and the sidewall **310,** but medical fluid **F** cannot substantially pass between the shuttle member **360** and the sidewall **310.** In some embodiments, the shuttle member **360** may include one or more apertures having sufficient cross-sectional area to allow the passage of air but substantially prevent the passage of medical fluid **F.**

The closure **330** is adhered to an aperture **331** at the distal end **314** of the prime tube **300** and may act as a stop to prevent the shuttle member **360** from being discharged out the distal end **314** of the prime tube **300.** The closure **330** may include an aperture **332** to allow air to exit the distal end **314** of the prime tube **300.** Various embodiments may include a lip or protrusion at the distal end **314** of the sidewall **310** instead of a closure **330,** where the inner diameter of the distal end **314** is less than the inner diameter of the sidewall **310** to prevent the shuttle member **360** from being discharged out the distal end **314** of the prime tube **300.**

During a priming/purging operation of the syringe **10A, 10B,** air passes through or around the shuttle member **360,** through the internal chamber **350,** and out the aperture **332** of the closure **330.** In some embodiments, air may pass through or around the shuttle member **360** without substantially dislodging and/or moving the shuttle member **350** within the internal chamber **350.** As the air is continued to be primed, eventually some medical fluid **F** pushes the remaining air from the syringe **10A, 10B** into the internal chamber **350,** and the medical fluid **F** itself enters the internal chamber **350.** The pressurized medical fluid **F** dislodges the shuttle member **360** from the frictional fit with the sidewall **310** and causes the shuttle member **360** to slide distally within the internal chamber **350** toward the closure **330.** As medical fluid **F** continues to be injected into the internal chamber **350** from the syringe **10A, 10B,** the shuttle member **360** may continue sliding distally until the shuttle member **360** contacts the closure **330** and substantially all of the air has been forced out of the internal chamber **350** by the pressurized medical fluid **F.**

With continued reference to **FIGS. 8A-9C****,** the sidewall **310** may be made of a translucent or transparent material such that the shuttle member **360** is visible through the sidewall **310.** The shuttle member **360** may be a readily visualized, conspicuous color, such that the movement of the shuttle member **360** along the longitudinal axis of internal chamber **350** may be observed or detected. Once a predetermined volume of the medical fluid **F** has entered the prime tube **300,** as evidenced by the longitudinal position of the shuttle member **360** within the internal chamber **350,** the technologist may determine that the syringe **10A, 10B** has been fully primed. In some embodiments, the internal chamber **350** may be sized such that the shuttle member **360** contacts the closure **330** (as shown in **FIG. 8D**) once a volume of medical fluid **F** sufficient to prime the syringe **10A, 10B** has been ejected into the internal chamber **350.** In such embodiments, a ceasing of movement of the shuttle member **360,** indicating that the shuttle member **360** has engaged the closure **330,** may be observed by the technologist to establish that the syringe **10A, 10B** has been fully primed.

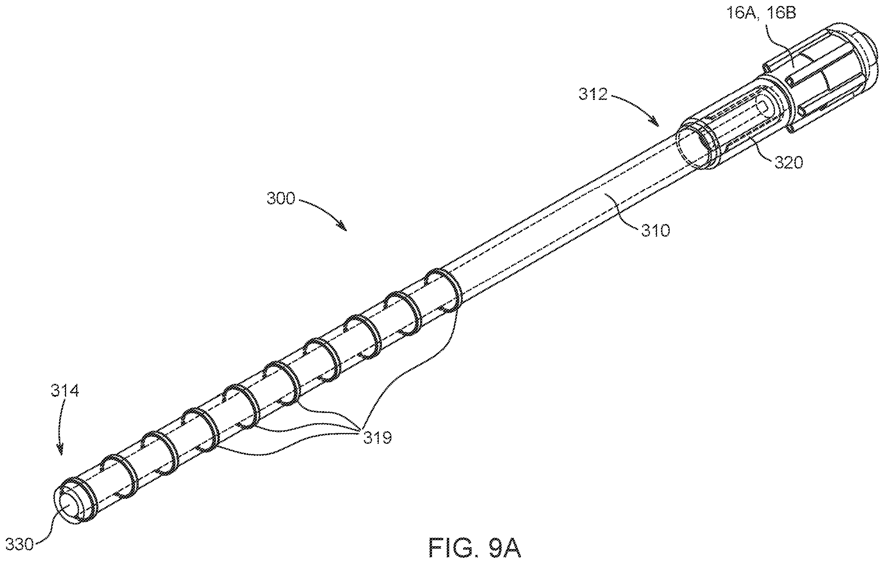

In some embodiments, the priming status sensor **910** (see **FIGS. 2B** and **3**) may be configured to detect the position of the shuttle member **360** within the internal chamber **350.** Referring specifically to **FIGS. 9A-9C****,** the sidewall **310** may include or more ribs **319** or other demarcations or indicators that the technologist and/or the priming status sensor **910** may use as a scale to assist in determining relative position and distance travelled of the shuttle member **360** within the internal chamber **350** and thus, the volume of medical fluid **F** that has entered the prime tube **300.** The priming status sensor **910** may include a detector for image recognition, such as a camera as described in U.S. Patent No. 10,201,666. Based on an output signal from the priming status sensor **910,** the controller **900** may be programmed or configured to determine that the shuttle member **360** has moved a sufficient distance indicating that the syringe **10A, 10B** is primed of air. The processor **900** may be configured to prevent a fluid injection procedure until it receives indication that the syringe **10A, 10B** has been successfully primed. Once a successful prime has been established as determined by the controller **900** and/or technologist, the prime tube **300** may be removed and discarded according to hospital protocol, and a suitably primed patient catheter line may be installed on the syringe **10A, 10B.** The prime tube **300** may include a one-way check valve at the proximal end to prevent fluid leakage upon removal.

In some embodiments, the aperture **332** of the closure **330** may be sized or have flow features configured to emit an audible sound, such as a whistle, when air flows through the aperture **332.** The technologist may determine that the syringe **10A, 10B** has been primed when the aperture **332** ceases to emit a sound, indicating that substantially all of the air has been expelled from the internal chamber **350.** Alternatively, the priming status sensor **910** may include a microphone configured to detect the sound emitted from the aperture **332.** The controller **900** may be programmed or configured to determine that the syringe **10A, 10B** has been primed when the aperture **332** ceases to emit a sound, indicating that substantially all of the air has been expelled from the internal chamber **350.**

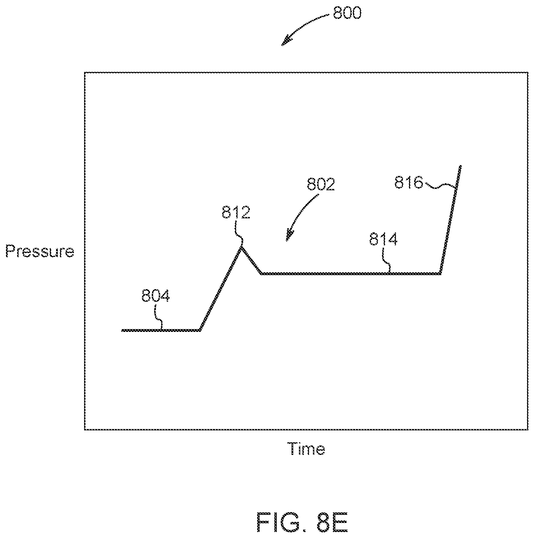

Referring now to **FIG. 8E****,** a graph 8**00** illustrating fluid pressure within the prime tube **300** against time is shown for the embodiment of **FIGS. 8A-8D****.** The fluid pressure, represented by curve **802,** is initially substantially constant at section **804** as primarily air is injected into the internal chamber **350** and flows through or around the shuttle member **360.** The fluid pressure then increases as medical fluid **F** enters the internal chamber **350,** until the fluid pressure reaches a sufficient pressure to dislodge the shuttle member **360** from the proximal end **314.** Dislodgement of the shuttle member **360** may cause a spike **812** in pressure as the initial static friction between the shuttle member **360** and the sidewall **310** is overcome. The pressure may then stabilize at section **814** as the shuttle member **360** is displaced by the medical fluid **F** and slides along the prime tube **300** until it reaches the distal end **314.** Once the shuttle member **360** contacts the distal end, fluid pressure builds quickly in the internal chamber **350,** as indicated by section **816.**

In some embodiments, the priming status sensor **910** may include a motor current (or motor force) sensor configured to measure the current draw of the drive motor associated with the piston **13A, 13B.** The controller **900** may be programmed or configured to detect changes in the fluid pressure based on changes in motor current. In particular, changes in motor current may correlate to changes in fluid pressure within the prime tube **300,** as shown in the graph of **FIG. 8E****.** For example, when the pressure stabilizes at section **814** or rises dramatically at section **816,** the controller **900** may determine that the air has been primed from the syringes and provide an indication that the priming operation is complete

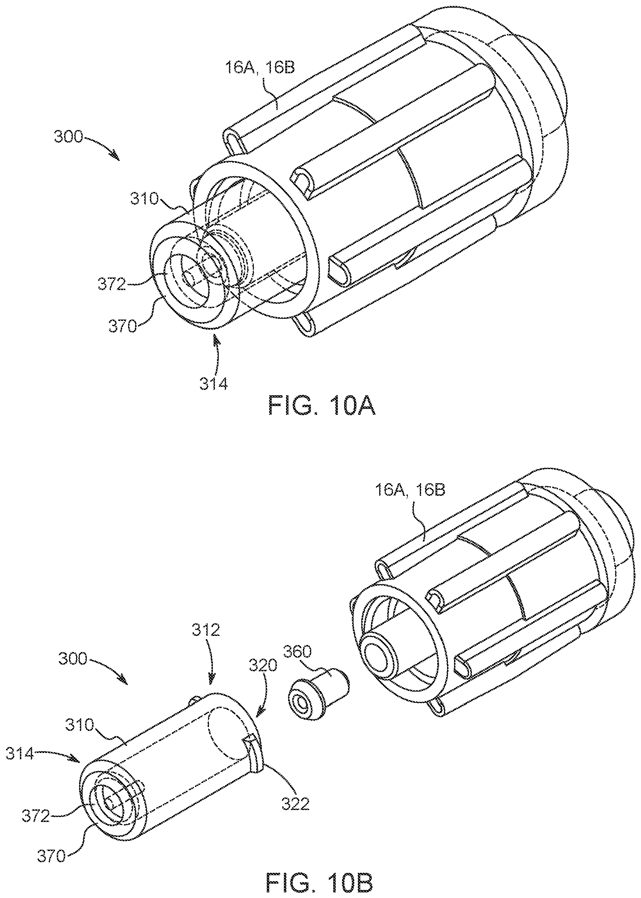

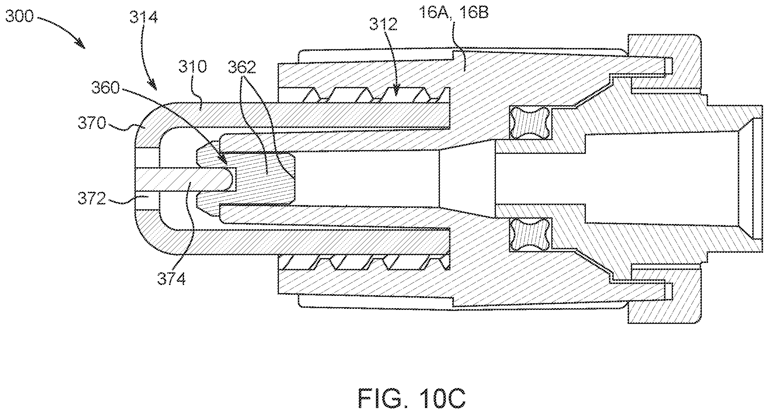

Referring now to **FIGS. 10A-10C****,** an embodiment of a prime tube **300** is shown in which the sidewall **310** defines a cap **370** at the distal end **314** having one or more air release holes **372** and a plug retention member **374.** A shuttle member **360** in the form of a porous plug is disposed within the cap **370,** and in an initial state or position of the prime tube **300** is held in the outlet **16A, 16B** of the syringe **10A, 10B** by the plug retention member **374.** In some embodiments, the shuttle member **360** may be made from a hydrophobic medical grade plastic, as described herein, that are permeable to air but impermeable to the medical fluid **F.** In some embodiments, the shuttle member **360** may be a solid material defining one or more apertures having a sufficient cross-sectional area to allow the passage of air but an insufficient cross-sectional area to allow the passage of the medical fluid **F.**

During a priming/purging operation, air ejected from the syringe **10A, 10B** flows through the porous shuttle member **360,** into the internal chamber **350,** and out the one or more air release holes **372.** As the air is continued to be primed and purged out of the syringe **10A, 10B,** eventually some medical fluid **F** pushes the remaining air from the syringe **10A, 10B** through the shuttle member **360** and the medical fluid **F** contacts a proximal face **362** of the shuttle member **360.** As the medical fluid **F** is unable to pass through the shuttle member **360,** fluid pressure builds against the proximal face **362** causing the shuttle member **360** to move to a second, primed position and bear against the plug retention member **374.**

In some embodiments, the controller **900** may be configured to measure the fluid pressure at the proximal face **362** of the shuttle member **360.** In particular, the priming status sensor **910** may include a motor current (or motor force) sensor configured to measure the current draw of the drive motor associated with the piston **13A, 13B.** The controller **900** may be programmed or configured to determine the fluid pressure based on motor current draw. The controller **900** may be programmed or configured to determine that the syringe **10A, 10B** has been primed when a predetermined fluid pressure is measured. Once a successful prime has been established by the controller **900,** the prime tube **300** may be removed and discarded according to hospital protocol, and a suitably primed patient catheter line may be installed on the syringe **10A, 10B.** One feature of the prime tube **300** illustrated in **FIGS 10A-10C** is that the volume of medical fluid **F** ejected during the priming operation is minimized, reducing hospital waste and saving on costs. Once the controller **900** has determined that the syringe has been primed, the controller may release the pressure on the piston **13A, 13B** and depressurize the fluid in the syringe to ensure that pressurized fluid is not ejected from the syringe when the prime tube **300** is removed.

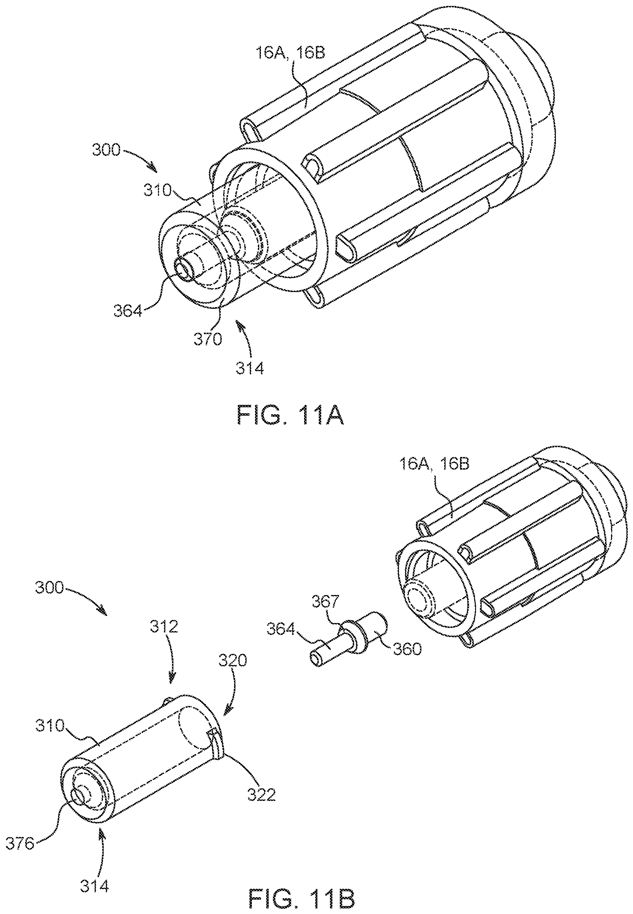

Referring now to **FIGS. 11A-11D****,** an embodiment of a prime tube **300** having a distally extending indicator tab is illustrated. The sidewall **310** defines a cap **370** at the distal end **314** having a receiving aperture **376.** The sidewall **310** engages with a threaded connector of an outlet **16A, 16B** of the syringe **10A, 10B** by a threadable lug **320.** A shuttle member **360** in the form of a porous plug is disposed within the internal chamber **350,** and in an initial state or position of the prime tube **300** the shuttle member **360** is held in the outlet **16A, 16B** of the syringe **10A, 10B** by a press fit. In some embodiments, the shuttle member **360** may be made from a hydrophobic medical grade plastic, as described herein, that are permeable to air but impermeable to the medical fluid **F.** In some embodiments, the shuttle member **360** may be a solid material defining one or more apertures having a sufficient cross-sectional area to allow the passage of air but an insufficient cross-sectional area to allow the passage of the medical fluid **F.** In certain embodiments, the shuttle member **360** may be colored a conspicuous color to be readily viewed by the technologist. The shuttle member **360** includes a distally extending tip **364** axially aligned with the receiving aperture **376** of the cap **370.** The diameter of the receiving aperture **376** may be slightly larger than the diameter of the distally extending tip **364** to allow air to pass between the space between the diameters.

During a priming/purging operation, air ejected from the syringe **10A, 10B** flows through the porous shuttle member **360,** into the internal chamber **350,** and out the receiving aperture **376.** As the air is continued to be primed and purged out of the syringe **10A, 10B,** eventually some medical fluid **F** pushes the remaining air from the syringe **10A, 10B** through the shuttle member **360,** and the medical fluid **F** contacts the proximal face **362** of the shuttle member **360.** As the medical fluid **F** is unable to pass through the shuttle member **360,** fluid pressure builds against the proximal face **362** causing the shuttle member **360** to be dislodged from the outlet **16A, 16B** of the syringe **10A, 10B** to a second, primed position and bear against the cap **370.** The tip **364** extends through the receiving aperture **376,** providing an indication to the technologist and/or the controller **900** that the shuttle member **360** has been dislodged and the syringe **10A, 10B** has been primed. A distal surface **367** of the shuttle member **360** may sealably abut an inner surface surrounding the receiving aperture **376** to prevent any medical fluid from escaping from the prime tube **300** once it has been primed.

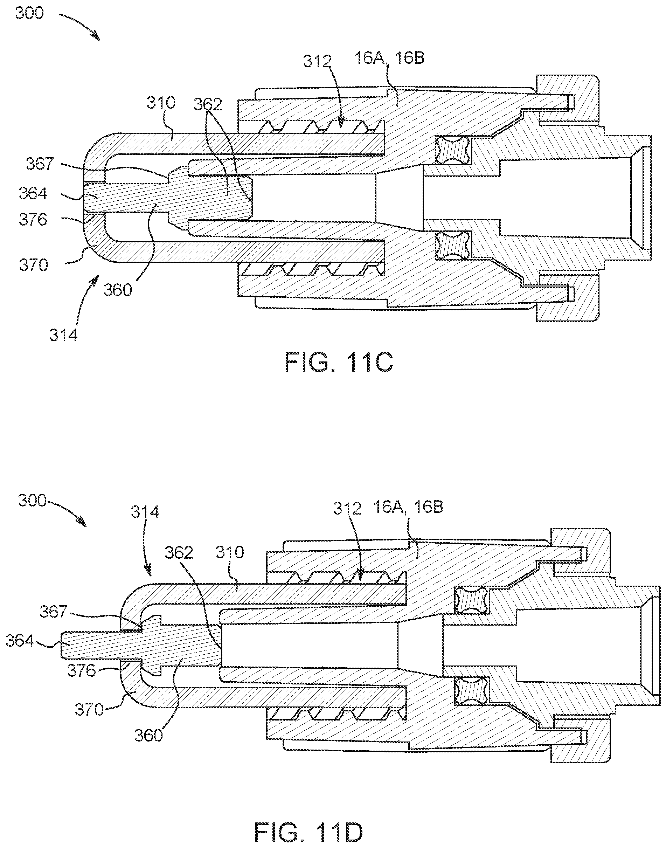

In some embodiments, the priming status sensor **910** may include a detector for image recognition, such as a camera as described in U.S. Patent No. 10,201,666. Priming status sensor **910** may be configured to detect movement and/or position of the tip **364** from an initial position in which tip **364** is flush with or recessed in the sidewall **310** (see **FIG. 11C**) to a primed position (see **FIG. 11D**) in which shuttle member **360** moves to the distal end **314 of** the prime tube **300** and tip **364** extends distally through the receiving aperture **376.** In some embodiments, priming status sensor **910** may include a limit switch contacted by the tip **364** when shuttle member **360** moves to the distal end **314** of prime tube **300.**

Based on an output signal from the priming status sensor **910,** the controller **900** may be programmed or configured to determine that the shuttle member **360** has moved to the distal end **314** of the prime tube **300,** indicating that the syringe **10A, 10B** has been primed. The processor **900** may be configured to prevent a fluid injection procedure until it receives indication that the syringe **10A, 10B** has been successfully primed. Once a successful prime has been established as determined by the controller **900** and/or technologist, the prime tube 300 may be removed and discarded according to hospital protocol, and a suitably primed patient catheter line may be installed on the syringe **10A, 10B.**

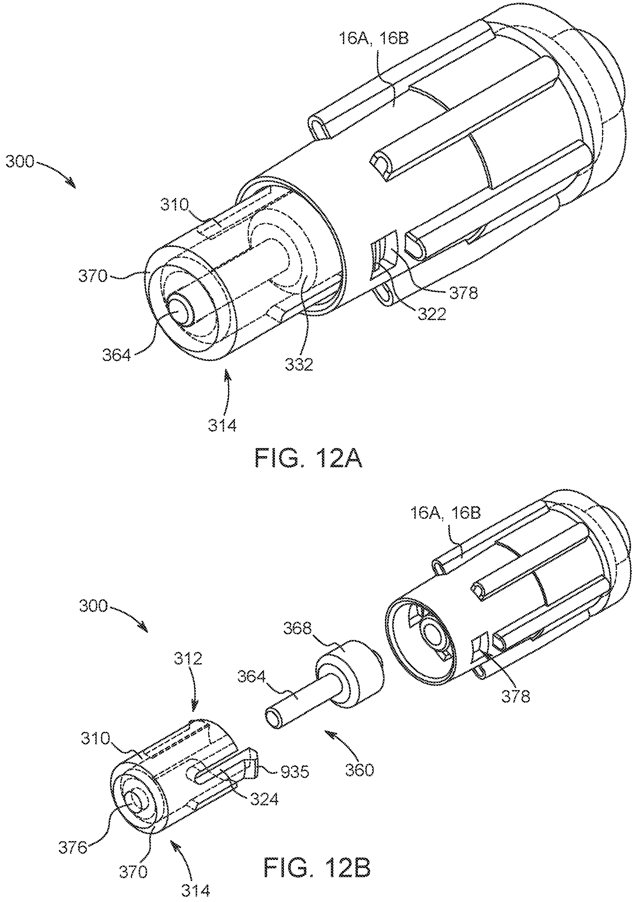

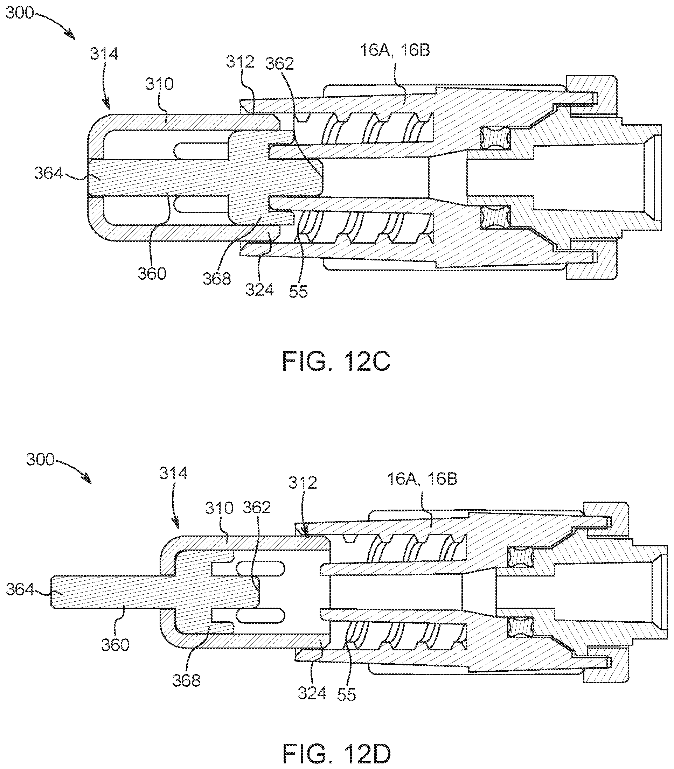

Referring now to **FIGS. 12A-12C****,** an embodiment of a prime tube **300** is similar to the embodiment of **FIGS. 11A-11D****,** and the primary differences will be discussed. In the embodiment shown in **FIGS. 12A-12C****,** the engaging lugs **322** are provided on deflectable arms **324** of the connector **320.** The engaging lugs **322** may, in some embodiments, include tabs that fit into corresponding slots **378** in the outlet **16A, 16B** of the syringe **10A, 10B.** The proximal end of the shuttle member **360** includes an at least partially circumferential wall **368** that surrounds an outer circumference of the outlet **16A, 16B** and engages the deflectable arms **324** in the initial position of the prime tube **300** (shown in **FIG. 12C**) to prevent inward deflection of the arms **324.** Engagement of the circumferential wall **368** with the deflectable arms **324** reversibly locks the engaging lugs **322** of the prime tube **300** in the corresponding slots **378** the outlet **16A, 16B** of the syringe **10A, 10B,** and prevents manual removal of the connector **320** of the prime tube **300** from the outlet **16A, 16B** of the syringe **10A, 10B** when the shuttle member **360** is sealed in the outlet **16A, 16B.** As before, the shuttle member **360** is permeable to air and impermeable to liquid, so as fluid pressure builds, the shuttle member **360** is dislodged from the outlet **16A, 16B.** When the shuttle member **360** moves distally to the primed position, the circumferential wall **368** disengages from between the outer circumference of the outlet **16A, 16B** and the deflectable arms **324** (as shown in **FIG. 12D**), allowing the arms **324** to deflect radially inward such that the engagement lugs **322** can release from the slots **378.** The prime tube **300** can then be removed from the syringe **10A, 10B.** Thus, the arrangement of the circumferential wall **368** and the deflectable arms **324** forces the technologist to perform a priming operation on the syringe **10A, 10B** before the prime tube **300** can be removed from the syringe **10A, 10B** ensuring that the syringes are primed before the injection procedure can proceed (i.e., by removal of the prime tubes **300)**. As before, the technologist and/or processor **900** can determine the status of the system (primed or unprimed) by the tip **364** extending through receiving aperture **376** and by the technologist being able to remove prime tube **300.**

In some embodiments, the engaging lugs **322** of the connector **320** may be configured to automatically release from the slots **378** of the outlet **16A, 16B** of the syringe **10A, 10B** at a predetermined fluid pressure corresponding to a fluid pressure at which the syringe **10A, 10B** is fully primed. Thus, release of the prime tube **300** from the syringe **10A, 10B** indicates that the syringe **10A, 10B** is primed and ready for an injection protocol. In some embodiments, the connector **320** may be configured so as to not be manually removable from the outlet **16A, 16B** of the syringe **10A, 10B,** thereby forcing the technologist to perform a priming operation to automatically release the prime tube **300** from the syringe **10A, 10B.** The syringe **10A, 10B** may be oriented such that the prime tube **300** falls into a waste container when the engaging lugs **322** release from the outlet **16A, 16B** of the syringe **10A, 10B.** A suitably primed patient catheter line may be installed on the syringe **10A, 10B** after the prime tube **300** has been released.

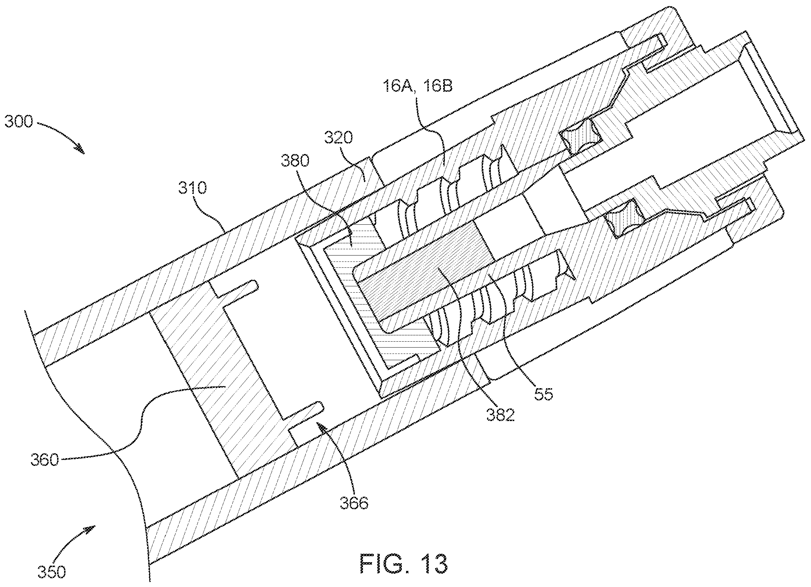

Referring now to **FIG. 13****,** an embodiment of a prime tube **300** including an inner cap **380** that fits over the inner nozzle **55** of the outlet **16A, 16B** of the syringe **10A, 10B** is illustrated. The cap **380** is configured and/or arranged to be recessed into the outlet **16A, 16B** so that the cap **380** cannot be manually removed from the inner nozzle **55** prior to the syringe being primed and can only be removed by a priming operation. Thus, an injection procedure cannot be performed without initial priming and resultant removal of the cap **380.** For example, the inner cap **380** may be recessed by a friction fit within the outlet **16A, 16B** so that a person cannot deliberately or inadvertently remove the inner cap **380** with ordinary effort. In some embodiments, the inner cap **380** may include a proximal post **382** that inserts into at least a portion of the inner nozzle **55** of the syringe **10A, 10B.** The proximal post **382** may help maintain the inner cap **380** in place on the inner nozzle **55** via a press fit or friction fit. In some embodiments, the inner cap **380** may be made from a hydrophobic medical grade plastic, as described herein, that is permeable to air but impermeable to the medical fluid **F.** In some embodiments, the inner cap **380** may be a solid material defining one or more apertures having a sufficient cross-sectional area to allow the passage of air but an insufficient cross-sectional area to allow the passage of the medical fluid **F.**

According to certain embodiments, a shuttle member **360** may be disposed and slidable within the interior chamber **350** of prime tube **300** and has a sufficiently tight fit to the sidewall **310** to prevent medical fluid **F** from flowing between the shuttle member **360** and the sidewall **310.** In some embodiments, the shuttle member **360** may include fingers **366** configured to engage the cap **380.** In some embodiments, the shuttle member **360** may be made from a hydrophobic medical grade plastic, as described herein, that is permeable to air but impermeable to the medical fluid **F.** In some embodiments, the shuttle member **360** may be a solid material defining one or more apertures having a sufficient cross-sectional area to allow the passage of air but an insufficient cross-sectional area to allow the passage of the medical fluid **F.** The distal end **314** of the sidewall **310** has at least one opening to allow air flowing through the cap **380** and the shuttle member **360** to exit the prime tube **300.**

During a priming operation, air ejected from the syringe **10A, 10B** flows through or around the inner cap **380** and the shuttle member **360,** into the internal chamber **350,** and out the distal end **314** of the prime tube **300.** As the air is continued to be primed and purged out of the syringe **10A, 10B,** eventually some medical fluid **F** pushes the remaining air from the syringe **10A, 10B** through the inner cap **380,** and the medical fluid **F** contacts the proximal surface or the proximal post **382** of the inner cap **380.** As the medical fluid **F** is unable to pass through the cap **380,** fluid pressure builds until inner cap **380** is dislodged from nozzle **55.** The inner cap **380** then slides distally within internal chamber **350** under the force of the medical fluid **F** as more fluid **F** is injected into the prime tube **300.** The inner cap **380** further engages and pushes the shuttle member **360** within the internal chamber **350** toward the distal end **314.**

Similar to embodiments discussed in connection with **FIGS. 8A-9C****,** the shuttle member **360** and/or the inner cap **380** may be a conspicuous color and the sidewall **310** may be at least partially transparent or translucent so that the shuttle member **360** and/or the inner cap **380** are visible within the internal chamber **350.** The technologist and/or the controller **900** may determine that the syringe **10A, 10B** has been primed based on the position of the shuttle member **360** and/or the inner cap **380** is visible within the internal chamber **350,** in substantially the same manner discussed herein in connection with **FIGS. 8A-9C****.**

In some embodiments, the syringe **10A, 10B** may be provided with the inner cap **380** already in place. As such, the inner cap **380** may help maintain a sterility of the interior of the syringe **380.** Further, as described herein due to the non-manually removable interface between the inner cap **380** from the inner nozzle **55,** the inner cap **380** may only be readily removed by a priming operation, thereby ensuring that the syringe **10A, 10B** is primed prior to connection of a patient catheter line and minimizing the occurrence of inadvertent injection of air during the injection procedure.

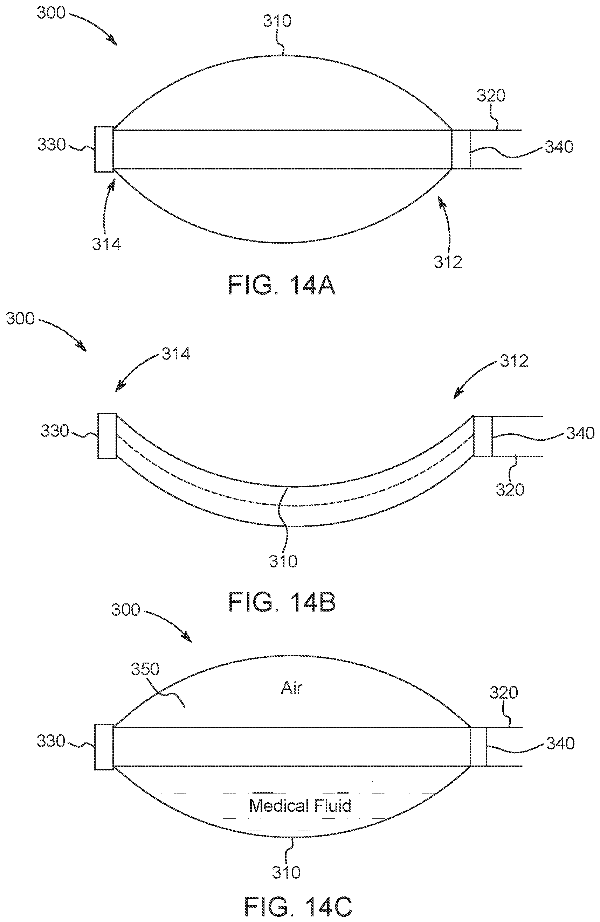

Referring now to **FIGS. 14A-14C****,** an embodiment of a prime tube **300** including an expandable body is illustrated. Similar to the embodiment discussed herein in connection with **FIGS. 4A-4D****,** a closure **330** in the form of a sealing plug may be inserted or otherwise adhered to the distal end **314** of the prime tube **300.** The closure **330** may prevent all fluid, including air and medical fluid **F,** from exiting the distal end **314** of the prime tube **300.** In some embodiments, the sidewall **300** may be made of a material which may be blow molded in an expanded state (shown in **FIG. 14A**) and then converted to a contracted or compressed state (shown in **FIG. 14B**), which is the state in which the prime tube **300** is supplied. When in the contracted state, the sidewall **310** is at least partially collapsed in on itself such that the volume of the internal chamber **350** is minimized. As shown in **FIG. 14A****,** an embodiment of the prime tube **300** is generally shaped as an ellipsoidal shell as manufactured although other molded shapes are envisioned. After manufacturing, a portion of the sidewall **310** is inverted such that opposite faces of the sidewall **310** touch or come into close proximity with one another. The sidewall **310** as shown, is thus shaped as a half ellipsoidal shell in the contracted state of **FIG. 14B****.** When pressure within the internal chamber **350** exceeds the pressure of the external environment and the compression pressure of the contracted sidewall **310,** at least a portion of the sidewall **310** is configured to revert such that the sidewall **310** assumes the expanded state, increasing the volume of the internal chamber **350.** In some embodiments, the prime tube **300** may further include a one-way valve **340** that prevents backflow from internal chamber **350** back out the proximal end **312** of the prime tube **300.**

The prime tube **300** according to the embodiment of **FIGS. 14A-14C** operates similarly to the embodiment described in connection with **FIGS. 5A-5C****.** Due to the sealed nature of the sidewall **310** and the closure **330,** air is trapped in the internal chamber **350** during the priming operation. As the air is continued to be primed, eventually some medical fluid **F** pushes the remaining air into the prime tube **300** and the medical fluid **F** itself enters the internal chamber **350.** As the volume of air and medical fluid **F** in the internal chamber **350** increases, the sidewall **310** inverts to accommodate the increased fluid volume and corresponding pressure. Once the volume of the internal chamber **350** reaches a specific size, the technologist may determine that the syringe **10A, 10B** has been primed and is ready for an injection procedure. In some embodiments the priming status sensor **910** may include a camera, as described herein in connection with **FIGS. 4A-4D****,** in conjunction with the controller **900** to determine that the syringe **10A, 10B** has been primed based on the sidewall **310** being in the expanded state. As described herein in connection with at least **FIGS. 4A-4D****,** the controller **900** may be programmed or configured to prevent performance of an injection procedure until a successful prime of the syringe **10A, 10B** has been detected.

During removal of the prime tube **300** from the syringe **10A, 10B,** the one-way valve **340** prevents the pressurized air and fluid in the internal chamber **150** from releasing from the proximal end **312** of the prime tube **300.** The entire prime tube **300** with air and medical fluid **F** contained therein may be discarded according to hospital protocol.

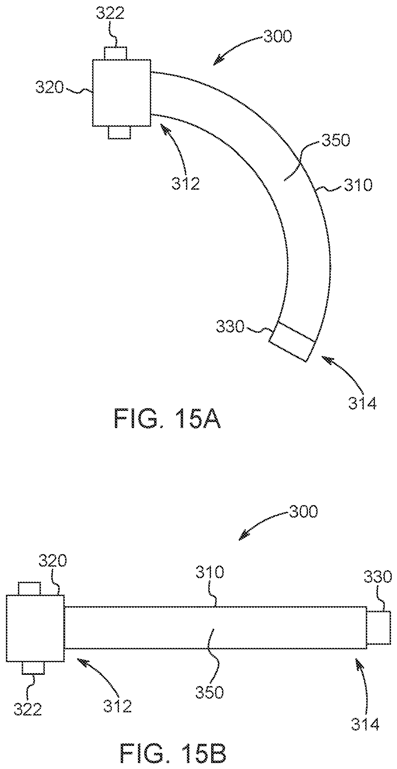

Referring now to **FIGS. 15A** and **15B****,** in some embodiments of the prime tube **300,** the sidewall **310** may be naturally in a bent, floppy, or flaccid state or configuration, resembling a flattened tube as shown in **FIG. 15A****.** When fluid pressure builds in the internal chamber **350,** such when medical fluid **F** flows through the sidewall **310,** the fluid pressure causes the sidewall **310** to straighten/extend and expand to the shape of a tube, as shown in **FIG. 15B****.** In some embodiment, the sidewall **310** may become substantially rigid in response to fluid flow through the internal chamber **350.** In other embodiments, the sidewall **310** may be in a flattened, rolled or coiled state in the absence of fluid flow in the internal chamber **350.** The sidewall **310** may transition to a straightened expanded shape due to fluid pressure when fluid flows through the internal chamber **350**. The closure **330** connected to the distal end **314** of the sidewall **310** may be permeable to air but impermeable to medical fluid **F,** as described for example in connection with **FIGS. 4A-4D** herein. The technologist and/or processor **900,** as describe herein, may visually determine when the prime tube **300** is in the straightened state (**FIG. 15B**) and may determine that a priming operation has been completed and may proceed with the next step of the fluid injection procedure. In some embodiments, the closure **300** may define flow features to induce an audible sound where air passes through the closure **330.** The technologist may determine that the syringe **10A, 10B** has been primed when the aperture **332** ceases to emit a sound, indicating that substantially all of the air has been expelled from the internal chamber **350.** Alternatively, the priming status sensor **910** may include a microphone configured to detect the sound emitted from the aperture **332.** Controller **900** may be programmed or configured to determine that syringe **10A, 10B** has been primed when closure **330** ceases to emit a sound, indicating that substantially all of the air has been expelled from internal chamber **350.** Once a successful prime has been established as determined by controller **900** and/or technologist, prime tube **300** may be removed and discarded according to hospital protocol, and a suitably primed patient catheter line may be installed on syringe **10A, 10B.**

While various examples of the present disclosure were provided in the foregoing description, those skilled in the art may make modifications and alterations to these examples without departing from the scope of the disclosure. For example, it is to be understood that features of various embodiments herein may be adapted to other embodiments described herein. Accordingly, the description is intended to be illustrative rather than restrictive. The disclosure is defined by the appended claims, and all changes to the disclosure that fall within the meaning and the range of equivalency of the claims are to be embraced within their scope.

## Claims

1. A prime tube (300) for use with a fluid injector (2000), the prime tube (300) comprising:
a sidewall (310) defining an internal chamber (350) having an expandable volume;
a connecter (320) associated with a proximal end (312) of the sidewall (310) and configured to reversibly engage an outlet (16A, 16B) of a fluid reservoir (10A, 10B) containing a medical fluid (F); and
a closure (330) associated with a distal end (314) of the sidewall (310), the closure (330) permeable to air and substantially impermeable to the medical fluid (F),
wherein the expandable volume of the internal chamber (350) is configured to increase as the medical fluid (F) enters the internal chamber (350).

2. The prime tube (300) according to claim 1, wherein the sidewall (310) comprises at least one bellows (316), wherein each of the at least one bellows (316) is configured to transition from a contracted state to an expanded state in response to an increase in fluid pressure within the internal chamber (350),
wherein the volume defined by the internal chamber (350) is greater with the at least one of the at least one bellows (316) in the expanded state than in the contracted state,
preferably wherein the at least one bellows (316) is stable in both the contracted state and the expanded state,
more preferably wherein an axial length of the sidewall (310) is greater with the at least one bellows (316) in the expanded state than in the contracted state.

3. The prime tube (300) according to claim 1, wherein the sidewall (310) comprises an elastomeric material configured to expand the expandable volume in response to an increase in fluid pressure within the internal chamber (350).

4. The prime tube (300) according to claim 1, wherein the sidewall (310) is configured to transition from a contracted state to an expanded state in response to an increase in fluid pressure within the internal chamber (350),
wherein, in the contracted state, a distal portion (313) of the sidewall (310) is rolled over a proximal portion (311) of the sidewall (310),
wherein, in the expanded state, the distal portion (313) of the sidewall (310) is at least partially unrolled from the proximal portion (311) of the sidewall (310), and
wherein the volume defined by the internal chamber (350) is greater with the sidewall (310) in the expanded state than in the contracted state.

5. The prime tube (300) according to claim 4, wherein, in the contracted state, an inner surface (323) of the distal portion (313) of the sidewall (310) faces an inner surface (321) of the proximal portion (311) of the sidewall (310),
preferably wherein at least a portion of the sidewall (310) is configured to invert in response to an increase in fluid pressure within the internal chamber (350) to increase the volume of the internal chamber (350).

6. The prime tube (300) according to claim 1, wherein the sidewall (310) is in a flaccid or bent configuration in the absence of fluid flow through the internal chamber (350) and in a rigid, extended configuration when fluid flows through the internal chamber (350).

7. The prime tube (300) according to claim 1, wherein the sidewall (310) is in a rolled, coiled configuration in the absence of fluid flow through the internal chamber (350) and in an unrolled, extended configuration when fluid flows through the internal chamber (350).

8. The prime tube (300) according to any of claims 1-7, further comprising a check valve (340) associated with the proximal end (312) of the sidewall (310) and configured to prevent fluid flow out of the proximal end (312).

9. The prime tube (300) according to any of claims 1-6, wherein the closure (330) comprises a porous material.

10. The prime tube (300) according to any of claims 1-6, wherein the closure (330) defines at least one aperture (332) having a cross-sectional area sized to allow passage of air and substantially prohibit passage of a medical fluid through the at least one aperture (332),
optionally wherein the at least one aperture is configured to emit an audible sound when air flows through the at least one aperture (332).

11. The prime tube (300) according to any of claims 1-6, wherein the closure (330) comprises a high crack pressure valve.

12. A fluid injector system (2000), comprising:
at least one fluid reservoir (10A, 10B) configured for injecting a medical fluid (F);
a prime tube (300) according to any of claims 1 to 11; and
at least one processor (900) programmed or configured to determine a priming status of the prime tube (300).

13. The fluid injector system (2000) according to claim 12, wherein the at least one processor (900) is programmed or configured to determine the priming status of the prime tube (300) based on a measured fluid pressure in at least one of the prime tube (300) and the fluid reservoir (10A, 10B).

14. The fluid injector system (2000) according to claim 12 or 13, further comprising an actuator for injecting the medical fluid from the at least one reservoir (10A, 10B, wherein the at least one processor (900) is programmed or configured to determine the priming status of the prime tube (300) based on a measured current draw of the actuator.

15. The fluid injector system (2000) according to any of claims 12-14, wherein the at least one processor (900) is programmed or configured to determine the priming status of the prime tube (300) based on at least one of expansion of the prime tube (300), a shape change of the prime tube (300), a sound emitted from the priming tube (300), and a force reading on a drive motor of the fluid injector (2000) associated with delivering a fluid from the fluid reservoir (10A, 10B).

## Patentansprüche

1. Entlüftungsröhre (300) zur Verwendung mit einem Fluidinjektor (2000), wobei die Entlüftungsröhre (300) Folgendes umfasst:
eine Seitenwand (310), die eine Innenkammer (350) mit einem expandierbaren Volumen definiert,
einen Verbinder (320), der einem proximalen Ende (312) der Seitenwand (310) zugeordnet und dazu ausgestaltet ist, einen Auslass (16A, 16B) eines Fluidreservoirs (10A, 10B), das ein medizinisches Fluid (F) enthält, reversibel in Eingriff zu nehmen, und
einen einem distalen Ende (314) der Seitenwand (310) zugeordneten Verschluss (330), wobei der Verschluss (330) luftdurchlässig und für das medizinische Fluid (F) im Wesentlichen undurchlässig ist,
wobei das expandierbare Volumen der Innenkammer (350) dazu ausgestaltet ist, sich zu vergrößern, wenn das medizinische Fluid (F) in die Innenkammer (350) eintritt.

2. Entlüftungsröhre (300) nach Anspruch 1, wobei die Seitenwand (310) mindestens einen Balg (316) umfasst, wobei jeder des mindestens einen Balgs (316) dazu ausgestaltet ist, als Reaktion auf eine Erhöhung des Fluiddrucks in der Innenkammer (350) aus einem zusammengezogenen Zustand in einen expandierten Zustand überzugehen,
wobei das durch die innere Kammer (350) definierte Volumen, wenn sich der mindestens eine des mindestens einen Balgs (316) in dem expandierten Zustand befindet, größer ist als in dem zusammengezogenen Zustand, vorzugsweise wobei der mindestens eine Balg (316) sowohl im zusammengezogenen Zustand als auch im expandierten Zustand stabil ist,
bevorzugter wobei eine axiale Länge der Seitenwand (310), wenn sich der mindestens eine Balg (316) im expandierten Zustand befindet, größer ist als im zusammengezogenen Zustand.

3. Entlüftungsröhre (300) nach Anspruch 1, wobei die Seitenwand (310) ein elastomeres Material umfasst, das dazu ausgestaltet ist, das expandierbare Volumen als Reaktion auf eine Erhöhung des Fluiddrucks in der Innenkammer (350) zu expandieren.

4. Entlüftungsröhre (300) nach Anspruch 1, wobei die Seitenwand (310) dazu ausgestaltet ist, als Reaktion auf eine Erhöhung des Fluiddrucks in der Innenkammer (350) aus einem zusammengezogenen Zustand in einen expandierten Zustand überzugehen,
wobei ein distaler Abschnitt (313) der Seitenwand (310) in dem zusammengezogenen Zustand über einen proximalen Abschnitt (311) der Seitenwand (310) gerollt ist,
wobei der distale Abschnitt (313) der Seitenwand (310) in dem expandierten Zustand zumindest teilweise von dem proximalen Abschnitt (311) der Seitenwand (310) abgerollt ist, und
wobei das durch die innere Kammer (350) definierte Volumen, wenn sich die Seitenwand (310) in dem expandierten Zustand befindet, größer ist als in dem zusammengezogenen Zustand.

5. Entlüftungsröhre (300) nach Anspruch 4, wobei eine Innenfläche (323) des distalen Abschnitts (313) der Seitenwand (310) in dem zusammengezogenen Zustand einer Innenfläche (321) des proximalen Abschnitts (311) der Seitenwand (310) zugewandt ist,
vorzugsweise wobei mindestens ein Abschnitt der Seitenwand (310) dazu ausgestaltet ist, als Reaktion auf eine Erhöhung des Fluiddrucks in der Innenkammer (350) zu invertieren, um das Volumen der Innenkammer (350) zu erhöhen.

6. Entlüftungsröhre (300) nach Anspruch 1, wobei sich die Seitenwand (310) in einer schlaffen oder gebogenen Konfiguration befindet, wenn keine Fluidströmung durch die Innenkammer (350) vorliegt, und sich in einer starren, erweiterten Konfiguration befindet, wenn Fluid durch die Innenkammer (350) strömt.

7. Entlüftungsröhre (300) nach Anspruch 1, wobei sich die Seitenwand (310) in einer aufgerollten, gewickelten Konfiguration befindet, wenn keine Fluidströmung durch die Innenkammer (350) vorliegt, und sich in einer abgerollten, erweiterten Konfiguration befindet, wenn Fluid durch die innere Kammer (350) strömt.

8. Entlüftungsröhre (300) nach einem der Ansprüche 1 - 7, ferner umfassend ein Rückschlagventil (340), das dem proximalen Ende (312) der Seitenwand (310) zugeordnet und dazu ausgestaltet ist, eine Fluidströmung aus dem proximalen Ende (312) zu verhindern.

9. Entlüftungsröhre (300) nach einem der Ansprüche 1 - 6, wobei der Verschluss (330) ein poröses Material umfasst.

10. Entlüftungsröhre (300) nach einem der Ansprüche 1 - 6, wobei der Verschluss (330) mindestens eine Öffnung (332) definiert, die eine Querschnittsfläche aufweist, die so bemessen ist, dass sie einen Durchgang von Luft gestattet und einen Durchgang eines medizinischen Fluids durch die mindestens eine Öffnung (332) im Wesentlichen verhindert,
optional wobei die mindestens eine Öffnung dazu ausgestaltet ist, einen hörbaren Ton zu emittieren, wenn Luft durch die mindestens eine Öffnung (332) strömt.

11. Entlüftungsröhre (300) nach einem der Ansprüche 1 - 6, wobei der Verschluss (330) ein Ventil mit hohem Öffnungsdruck umfasst.

12. Fluidinjektorsystem (2000), umfassend:
mindestens ein zum Injizieren eines medizinischen Fluids (F) ausgestaltetes Fluidreservoir (10A, 10B),
eine Entlüftungsröhre (300) nach einem der Ansprüche 1 bis 11 und
mindestens einen Prozessor (900), der zum Bestimmen eines Entlüftungszustands der Entlüftungsröhre (300) programmiert oder ausgestaltet ist.

13. Fluidinjektorsystem (2000) nach Anspruch 12, wobei der mindestens eine Prozessor (900) dazu programmiert oder ausgestaltet ist, den Entlüftungszustand der Entlüftungsröhre (300) auf der Grundlage eines gemessenen Fluiddrucks in der Entlüftungsröhre (300) und/oder dem Fluidreservoir (10A, 10B) zu bestimmen.

14. Fluidinjektorsystem (2000) nach Anspruch 12 oder 13, ferner umfassend einen Aktuator zum Injizieren des medizinischen Fluids aus dem mindestens einen Reservoir (10A, 10B), wobei der mindestens eine Prozessor (900) dazu programmiert oder ausgestaltet ist, den Entlüftungszustand der Entlüftungsröhre (300) auf der Grundlage einer gemessenen Stromaufnahme des Aktuators zu bestimmen.

15. Fluidinjektorsystem (2000) nach einem der Ansprüche 12 - 14, wobei der mindestens eine Prozessor (900) dazu programmiert oder ausgestaltet ist, den Entlüftungszustand der Entlüftungsröhre (300) auf der Grundlage der Expandierung der Entlüftungsröhre (300) und/oder einer Formänderung der Entlüftungsröhre (300) und/oder eines von der Entlüftungsröhre (300) emittierten Tons und/oder eines Kraftmesswerts an einem Antriebsmotor des Fluidinjektors (2000), der dem Zuführen eines Fluids aus dem Fluidreservoir (10A, 10B) zugeordnet ist, zu bestimmen.

## Revendications

1. Tube amorce (300) destiné à être utilisé avec un injecteur de fluide (2000), le tube amorce (300) comprenant :
une paroi latérale (310) définissant une chambre interne (350) ayant un volume extensible ;
un raccord (320) associé à une extrémité proximale (312) de la paroi latérale (310) et conçu pour venir en prise de manière réversible avec une sortie (16A, 16B) d'un réservoir de fluide (10A, 10B) contenant un fluide médical (F) ; et
une fermeture (330) associée à une extrémité distale (314) de la paroi latérale (310), la fermeture (330) étant perméable à l'air et sensiblement imperméable au fluide médical (F),
le volume extensible de la chambre interne (350) étant conçu pour augmenter lorsque le fluide médical (F) pénètre dans la chambre interne (350).

2. Tube amorce (300) selon la revendication 1, la paroi latérale (310) comprenant au moins un soufflet (316), chacun de l'au moins un soufflet (316) étant conçu pour passer d'un état contracté à un état dilaté en réponse à une augmentation de la pression de fluide à l'intérieur de la chambre interne (350),
le volume défini par la chambre interne (350) étant plus grand lorsque l'au moins un de l'au moins un soufflet (316) est à l'état dilaté qu'à l'état contracté, de préférence, l'au moins un soufflet (316) étant stable à la fois à l'état contracté et à l'état dilaté,
de manière davantage préférée, une longueur axiale de la paroi latérale (310) étant plus grande lorsque l'au moins un soufflet (316) est à l'état dilaté qu'à l'état contracté.

3. Tube amorce (300) selon la revendication 1, la paroi latérale (310) comprenant un matériau élastomère conçu pour dilater le volume extensible en réponse à une augmentation de la pression de fluide à l'intérieur de la chambre interne (350).

4. Tube amorce (300) selon la revendication 1, la paroi latérale (310) étant conçue pour passer d'un état contracté à un état dilaté en réponse à une augmentation de la pression de fluide à l'intérieur de la chambre interne (350),
à l'état contracté, une partie distale (313) de la paroi latérale (310) étant enroulée sur une partie proximale (311) de la paroi latérale (310),
à l'état dilaté, la partie distale (313) de la paroi latérale (310) étant au moins partiellement déroulée de la partie proximale (311) de la paroi latérale (310), et le volume défini par la chambre interne (350) étant plus grand lorsque la paroi latérale (310) est à l'état dilaté qu'à l'état contracté.

5. Tube amorce (300) selon la revendication 4, à l'état contracté, une surface intérieure (323) de la partie distale (313) de la paroi latérale (310) faisant face à une surface intérieure (321) de la partie proximale (311) de la paroi latérale (310),
de préférence, au moins une partie de la paroi latérale (310) étant conçue pour s'inverser en réponse à une augmentation de la pression de fluide à l'intérieur de la chambre interne (350) afin d'augmenter le volume de la chambre interne (350).

6. Tube amorce (300) selon la revendication 1, la paroi latérale (310) étant dans une configuration flasque ou courbée en l'absence d'écoulement de fluide à travers la chambre interne (350) et dans une configuration allongée rigide lorsque le fluide s'écoule à travers la chambre interne (350).

7. Tube amorce (300) selon la revendication 1, la paroi latérale (310) étant dans une configuration enroulée, en spirale en l'absence d'écoulement de fluide à travers la chambre interne (350) et dans une configuration déroulée, étendue lorsque le fluide s'écoule à travers la chambre interne (350).

8. Tube amorce (300) selon l'une quelconque des revendications 1 à 7, comprenant en outre un clapet anti-retour (340) associé à l'extrémité proximale (312) de la paroi latérale (310) et conçu pour empêcher un écoulement de fluide hors de l'extrémité proximale (312).

9. Tube amorce (300) selon l'une quelconque des revendications 1 à 6, la fermeture (330) comprenant un matériau poreux.

10. Tube amorce (300) selon l'une quelconque des revendications 1 à 6, la fermeture (330) définissant au moins une ouverture (332) ayant une aire de section transversale dimensionnée pour permettre le passage d'air et empêcher sensiblement le passage d'un fluide médical à travers l'au moins une ouverture (332), éventuellement, l'au moins une ouverture étant conçue pour émettre un son audible lorsque l'air s'écoule à travers l'au moins une ouverture (332).

11. Tube amorce (300) selon l'une quelconque des revendications 1 à 6, la fermeture (330) comprenant une soupape à haute pression de fissuration.

12. Système d'injection de fluide (2000), comprenant :
au moins un réservoir de fluide (10A, 10B) conçu pour injecter un fluide médical (F) ;
un tube amorce (300) selon l'une quelconque des revendications 1 à 11 ; et
au moins un processeur (900) programmé ou conçu pour déterminer un état d'amorçage du tube amorce (300).

13. Système d'injection de fluide (2000) selon la revendication 12, l'au moins un processeur (900) étant programmé ou conçu pour déterminer l'état d'amorçage du tube amorce (300) sur la base d'une pression de fluide mesurée dans au moins l'un du tube amorce (300) et du réservoir de fluide (10A, 10B).

14. Système d'injection de fluide (2000) selon la revendication 12 ou 13, comprenant en outre un actionneur pour injecter le fluide médical à partir de l'au moins un réservoir (10A, 10B), l'au moins un processeur (900) étant programmé ou conçu pour déterminer l'état d'amorçage du tube amorce (300) sur la base d'une consommation de courant mesurée de l'actionneur.

15. Système d'injection de fluide (2000) selon l'une quelconque des revendications 12 à 14, l'au moins un processeur (900) étant programmé ou conçu pour déterminer l'état d'amorçage du tube amorce (300) sur la base d'au moins un élément parmi une dilatation du tube amorce (300), un changement de forme du tube amorce (300), un son émis par le tube amorce (300), et une lecture de force sur un moteur d'entraînement de l'injecteur de fluide (2000) associée à la distribution d'un fluide à partir du réservoir de fluide (10A, 10B).
